(19) Europäisches Patentamt
European Patent Office
Office européen des brevets

(11) **EP 2 243 472 B1**

(12) **EUROPÄISCHE PATENTSCHRIFT**

(45) Veröffentlichungstag und Bekanntmachung des Hinweises auf die Patenterteilung:
**05.04.2017 Patentblatt 2017/14**

(51) Int Cl.:
*A61K 9/19* (2006.01)     *A61K 9/16* (2006.01)
*A61K 47/36* (2006.01)     *A61K 47/44* (2017.01)

(21) Anmeldenummer: **10160421.3**

(22) Anmeldetag: **20.04.2010**

(54) **Gefriergetrocknete Wirkstoffzusammensetzung**

Freeze-dried agent compound

Composition de matière active séchée par congélation

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **22.04.2009 EP 09158473**

(43) Veröffentlichungstag der Anmeldung:
**27.10.2010 Patentblatt 2010/43**

(73) Patentinhaber: **MedSkin Solutions Dr. Suwelack AG**
**48727 Billerbeck (DE)**

(72) Erfinder:
• **Kuhlmann, Fabian**
**48720, Rosendahl (DE)**
• **Elsinghorst, Claudia**
**48727, Billerbeck (DE)**
• **Wagner, Uwe**
**48308, Senden (DE)**
• **Malessa, Ralf, Dr.**
**45134, Essen (DE)**

(74) Vertreter: **CH Kilger Anwaltspartnerschaft mbB**
**Fasanenstrasse 29**
**10719 Berlin (DE)**

(56) Entgegenhaltungen:
EP-A- 0 888 769      WO-A-01/28600
WO-A-03/068843     WO-A-2004/035023
WO-A-2008/020066   DE-A1-102006 038 629
FR-A- 2 886 845

**Beschreibung**

Die Erfindung betrifft gefriergetrocknete Formkörper, enthaltend > 50 Gew. % eines oder mehrerer, ausgewählt aus der Gruppe der sauren Wirkstoffe mit einen pKs-Wert bei 25 °C von < 7, und ≤ 15 Gew. % eines oder mehrerer Gerüstbildner, wobei Proteine ausgenommen sind, wobei mindestens ein Gerüstbildner Chitosan ist, sowie gegebenenfalls einen oder mehrere Hilfsstoffe, jeweils bezogen auf die Gesamtzusammensetzung des gefriergetrockneten Formkörpers, deren 1 gewichtsprozentige Lösung oder Suspension in Wasser bei 20 °C einen pH Wert < 7 aufweist.

Weiterhin betrifft die Erfindung Verfahren zur Herstellung dieser gefriergetrockneten Formkörper, die Kombination solcher gefriergetrockneten Formkörper in Kit-of-parts Anordnungen zusammen mit wässrigen Lösungen sowie die Verwendung der gefriergetrockneten Formkörper und der Kit-of-parts Kombinationen zur pharmazeutischen und kosmetischen Anwendung.

Eine Reihe von wichtigen und hochpotenten Wirkstoffen für die kosmetische oder pharmazeutische Anwendung ist bekannt dafür, instabil zu sein und aufgrund äußerer Einflüsse derart verändert oder abgebaut zu werden, dass sie die erwünschte Wirkung in der Zusammensetzung, in der sie enthalten sind nicht mehr oder nicht mehr hinreichend erfüllen können, oder dass die veränderten Wirkstoffe bzw. deren abgebaute Nebenprodukte sogar eine schädliche Wirkung entfalten. Unter solchen labilen Wirkstoffen sind hier insbesondere solche Wirkstoffe zu nennen, die eine hohe Instabilität gegenüber thermischen Einflüssen aufweisen sowie solche, die hochgradig licht-, feuchtigkeits- und/oder oxidationsempfindlich sind.

Es besteht somit ein zentrales Interesse daran, solche hochpotenten und hochgradig instabilen und abbaugefährdeten Wirkstoffe in eine Form zu bringen, die eine hohe und langfristige Stabilität und damit einhergehend eine gute Lagerfähigkeit, optimale und reproduzierbare Wirkstoffgehalt-Bereitstellung über die gesamte Lager- und Verabreichungszeit und somit höchstmögliche Sicherheit und Effektivität bei der Verwendung ermöglichen.

Dabei ist neben einer wirksamen Stabilisierung der Wirkstoffe aber auch die Bereitstellung dieser in einer optimal geeigneten und auf den jeweiligen Anwendungszweck bestmöglich abgestimmten Darreichungsform von besonderem Interesse. Die Wahl der geeigneten Darreichungsform hängt dabei insbesondere ab von der Art und dem Ort der Applikation, der Zielgruppe und deren Eigenheiten, der Art und Höhe der Dosierung der Wirkstoffe oder ihrer Darreichungsform sowie beispielsweise auch den physikalischen und biochemischen Charakteristika der Wirkstoffe insbesondere in Hinblick auf ihre biologische Verfügbarkeit und systemische Wirkungsweise, die es hierbei zu beachten gilt.

Insbesondere Darreichungsformen für die äußere Anwendung sowie orale Applikationsformen sind hier für die Bereitstellung solcher stabilen hochpotenten Wirkstoffe von besonderem Interesse. Dabei sind für derartige Applikationen besonders geeignete und bevorzugte Darreichungsformen solche, die in wässrigen und/oder wasserhaltigen Formulierungen bzw. Umgebungen einsetzbar sind und in solchen wässrigen Milieus schnell-löslich sind. Dies ist insbesondere bei Wirkstoffsystemen für die orale Applikation von Bedeutung.

Um biologische oder organische Substanzen gegen feuchtigkeitsbedingten Abbau, Verderb oder Zersetzung zu schützen, ist die Methode der Gefriertrocknung bekannt und findet breite Anwendung. Hierbei werden die Substanzen häufig direkt der Gefriertrocknung unterworfen und insbesondere in der Pharmazeutik werden so die instabilen Wirkstoffe per se haltbar gemacht. Für die Bereitstellung der gefriergetrockneten, haltbaren Wirkstoffe in geeigneten pharmazeutischen oder auch kosmetischen Applikationsformen für die letztendliche Anwendung am oder im Körper stellt sich dann allerdings wiederum das Problem der stabilen und weiterhin feuchtigkeitsgeschützten Inkorporierung der gefriergetrockneten und somit stabilisierten Wirkstoffe in die gewünschte Darreichungsform. Trockene Darreichungsformen wie Pulvergemische, zu Tabletten verpresste Mischungen, in Kapseln gefüllte Wirkstoffe oder zu Granulaten verarbeitete Wirkstoffsysteme sind hinlänglich bekannt. Die mit solchen Applikationssystemen einhergehenden Nachteile sind insbesondere die häufig schlechte Löslichkeit und somit langsame Wirkstofffreisetzung, der hohe Anteil an unwirksamen jedoch für die Verarbeitung notwendigen Hilfs- und Füllstoffen, die in der Regel schlechte Eignung zur äußerlichen Anwendung sowie eine unzureichende Dosierbarkeit und damit unsichere Handhabbarkeit bzw. Probleme bei der Applikation durch den Endanwender.

Eine geeignete und bekannte Möglichkeit, Wirkstoffe in einer Applikations- oder Dosierform stabilisiert bereitzustellen besteht darin, die Wirkstoffe in einem System von Trägerstoffen zu dispergieren und diese Mischung der Gefriertrocknung zu unterziehen. Als Trägerstoffe werden hierbei zumeist solche Substanzen gewählt, die ein gutes Auflösungs- bzw. Quellverhalten aufweisen und dabei durch die Quellung eine gute Texturbildung ermöglichen, so dass das aufgelöste bzw. in dem Quellstoff dispergierte Wirkstoffsystem direkt als Applikationsform einsetzbar ist. Solche Systeme sind aufgrund der guten Löslichkeit bekannt und geeignet für die Bereitstellung von oralen Applikationsformen als auch für die Herstellung von kosmetischen oder pharmazeutischen Darreichungsformen für die äußerliche Anwendung. Hierbei ist verstärkt ein Interesse in der Bereitstellung von sogenannten single-unit Anwendungsformen vorhanden, was eine einfache und zielgenaue Wirkstoff-Dosis-Applikation für den Endanwender ermöglicht. Als single-unit Anwendungsformen werden hier Applikationssysteme verstanden, die im Gegensatz zu Pulvern oder Granulaten pro Anwendungseinheit die gewünschte und notwendige Wirkstoffmenge in einer einzigen Applikationseinheit enthalten wie beispielsweise Tabletten oder Kapseln, ohne dabei jedoch die Nachteile der schlechten Löslichkeit oder der fehlenden Eignung für die äußerliche Applikation mit sich zu führen.

Somit werden solche leicht-löslichen, durch Trocknung feuchtigkeitsstabilisierten single-unit Applikationsformen für die orale und/oder äußerliche Anwendung von instabilen Wirkstoffen vermehrt in Form größer formatiger Ausgestaltungen interessant, insbesondere wenn hohe Mengen von Wirkstoffen verabreicht werden sollen. Dabei besteht die besondere Herausforderung in der Regel darin, hohe Wirkstoffgehalte in schnell- und möglichst vollständig bzw. rückstandslos löslicher Form bereitzustellen, wobei ein möglichst hoher Wirkstoffgehalt bei einem möglichst geringen Anteil an Träger-oder Hilfsstoffen in der Zusammensetzung wünschenswert ist. Je geringer der Träger- bzw. Hilfsstoffanteil, desto rückstandsloser und vollständiger ist in der Regel die Auflösung, desto geringer ist dann allerdings in der Regel auch die mechanische Stabilität der Einzelformen, was insbesondere bei großformatigen Formkörpern für deren Verpackung, Lagerung und Handhabung eine Rolle spielt.

Die in solchen Applikationsformen üblicherweise verwendeten quellbaren oder löslichen Trägerstoffe werden zumeist ausgewählt aus Trägerstoffen, die insbesondere in einem neutralen bis alkalischen Milieu besonders gute Quellungs- und Löslichkeitseigenschaften aufweisen, in diesem pH-Bereich also besonders gut verarbeitbar sind. Außerdem werden für äußerlich anzuwendende Formulierungen üblicherweise neutrale bis leicht alkalische Formulierungen verwendet, da solche im allgemeinen über eine bessere Hautverträglichkeit verfügen und eine geringere Irritationsneigung zeigen. Dadurch ergibt sich aber speziell für die Gruppe der sauren Wirkstoffe, also solcher Wirkstoffe, die eine pH-abhängige Dissoziationsneigung aufweisen und damit unter neutralisierten bzw. alkalischen Bedingungen in dissoziierter Form vorliegen die Problematik, dass solche Systeme insbesondere bei hohen Wirkstoffgehalten nur bedingt einer Gefriertrocknung unterzogen werden können. Durch die hohe Dissoziationsneigung solcher Wirkstoffe bildet sich einerseits in der zu gefriertrocknenden Formulierung eine derart hohe Ionenkonzentration, dass eine Gefrierpunktserniedrigung der Zusammensetzung eintritt, die die Gefriertrocknungsbedingungen in unannehmbarem Maße erschwert. Weiter erhöht die hohe Ionenkonzentration im gefriergetrockneten Produkt dessen Hygroskopizität derart, dass insbesondere Formulierungen mit geringen Gehalten an Trägerstoffen, die maßgeblich die mechanische Stabilität der gefriergetrockneten Zusammensetzungen bedingen, nicht über eine ausreichende Stabilität verfügen. Bekannte und wichtige saure Wirkstoffe, die die genannten Eigenschaften aufweisen sind beispielsweise Ascorbinsäure (Vitamin C) und ihre Derivate sowie z. B. auch Acetylsalicylsäure (ASS) und ihre Derivate zu nennen.

Es besteht also die Notwendigkeit, gut dosierbare, großformatige single-unit Applikationsformen mit hoher Beladung instabiler, insbesondere saurer Wirkstoffe mit hoher pH-Wert abhängiger Dissoziationsneigung und geringem Anteil an unlöslichem oder quellbarem Trägerstoff und somit schneller und vollständiger Löslichkeit und höchstmöglicher mechanischer Stabilität für die kosmetische und pharmazeutische äußerliche und orale Verwendung bereitzustellen.

Dosierbare, wirkstoffbeladene Pellets oder single-unit Arzneiformen einer gewissen Größe, die die Bereitstellung hydrolyseempfindlicher Wirkstoffe wie z. B. Vitamine, insbesondere Vitamin C sowohl für die pharmazeutische als auch kosmetische Applikation ermöglichen sollen sind beschrieben in der US 5,405,616 und in der DE4201179. Die darin verwendeten Trägermaterialien sind vorzugsweise solche auf Basis von Proteinen. Die Pellets werden hergestellt, indem Dispersionen aus den Protein-Gerüstbildnern und gegebenenfalls kosmetischen oder pharmazeutischen Wirkstoffen in tiefkalte inerte Flüssigkeiten, vorzugsweise flüssigen Stickstoff, eingetropft werden und die gefrorenen Pellets anschließend abgetrennt und gefriergetrocknet werden. Um unter diesen Bedingungen Pellets zu bilden, ist jedoch die Anwesenheit von Protein-Gerüstbildnern, insbesondere Kollagen bzw. Kollagen-Derivaten erforderlich, da nur die genannten Protein-Gerüstbildner unter diesen Bedingungen stabile Pellets ausbilden können. Außerdem ist es mit dem hier beschriebenen Herstellverfahren nicht möglich, solche Formkörper herzustellen, die einen hohen Wirkstoffgehalt und lediglich einen geringen Anteil an Gerüstbildner aufweisen. Zwar wird das beschriebene Verfahren als besonders geeignet beschrieben, um insbesondere hydrolyseempfindliche Wirkstoffe wie z. B. Ascorbinsäure (Vitamin C) oder auch Acetylsalicylsäure verfügbar zu machen, allerdings sind die in den Beispielen beschriebenen Zusammensetzungen durchweg dadurch gekennzeichnet, dass der Gehalt an gerüstbildenden Bestandteilen um ein vielfaches höher ist, als der Anteil der Wirkstoffe. Das Verfahren ist somit nicht geeignet, Pellets mit im Vergleich zum gerüstbildenden Trägermaterial besonders hohen Wirkstoffkonzentrationen bereitzustellen. Auch dies hängt mit den besonderen Anforderungen an eine hohe Stabilisierung der Zusammensetzung im verwendeten Eintropfverfahren zusammen.

Eine ähnliche Problematik ergibt sich aus dem in der US 5,843,347 dargestellten Verfahren zur Herstellung poröser galenischer Partikel, die laut beschreibendem Teil in Größen bis 5 mm erhalten werden sollen. Bei dem hierin verwendeten Verfahren wird eine Mischung der Wirkstoffe in einer Matrix extrudiert und in Partikel der gewünschten Größe geschnitten, die anschließend durch Gefriertrocknung die porösen Formkörper bilden. Die Ausführungsbeispiele, zeigen jedoch lediglich, dass sogenannte Mikroperlen mit Durchmessern bis maximal 1,5 mm erhältlich sind. Dies ist auf die erfindungsgemäße Verwendung eines Extrusions- und Schneidverfahrens zurückzuführen, welches eine gewisse mechanische Stabilität der extrudierten Masse voraussetzt. Dies kann in der Regel durch einen relativ hohen Anteil an gerüstbildenden Polymeren bzw. Trägerstoffen und Stabilisatoren oder Füllstoffen erreicht werden. Werden jedoch lediglich geringe Mengen stabilisierender Träger- oder Hilfsstoffe im Vergleich zum Wirkstoffgehalt eingesetzt, so können, wie in diesem Dokument gezeigt, lediglich sehr kleinformatige Mikroperlen erhalten werden. Eine spezielle Möglichkeit zur Stabilisierung von instabilen, insbesondere sogenannten sauren Wirkstoffen ist nicht Gegenstand der Offenbarung.

Ein anderes Verfahren, das die Herstellung einer wirkstoffhaltigen Emulsion, Gießen dieser Emulsion in eine Form und anschließende Gefriertrocknung der Emulsion beinhaltet, ist Gegenstand der WO 05/073296. Die mit diesem Verfahren herstellbaren Formkörper sind nach den Ausführungen im beschreibenden Teil in Größen von 0,2 - 5 mm und größer erhältlich, löslich oder dispergierbar in Wasser und auch dieses Verfahren soll eine Möglichkeit zur Stabilisierung von instabilen Wirkstoffen wie z. B. Vitaminen zur Verfügung stellen. Gegenstand des vorliegenden Verfahrens ist jedoch primär die Bereitstellung von Formkörpern, worin die Wirkstoffe in einer Öl-in-Wasser-Emulsion eingearbeitet werden, die zur Herstellung dieser Emulsion zusätzlich Emulgatoren wie oberflächenaktive Substanzen enthält. Auch bei diesem Verfahren zeigt sich jedoch, dass die Einarbeitung von extrem hohen Wirkstoffgehalten bei geringen Gerüstbildnermengen aus Gründen der Stabilität der großformatigen Formkörper nicht möglich ist. So ist der Anteil der Gerüstbildner angegeben mit 10 bis 95 % bei einem Gehalt an bis zu 5 % oberflächenaktivem Mittel. Auch das Beispiel gibt keine Anhaltspunkte, dass das beschriebene Verfahren geeignet ist für die Herstellung von Formkörpern, die primär aus einem feuchtigkeitsinstabilen, insbesondere sauren Wirkstoff mit lediglich geringen Mengen stabilisierendem Polymer bestehen.

Auch Dokument JP 2004-149468 offenbart ein Verfahren zur Stabilisierung eines feuchtigkeitsinstabilen Wirkstoffs, L-Ascorbinsäure-2-phosphat, einem Ascorbinsäure-Derivat (Vitamin C-Derivat), durch Gefriertrocknung einer Wirkstoff-Gerüstbildner-Mischung unter Erhalt eines leicht-löslichen gefriergetrockneten Formkörpers für die kosmetische Behandlung. In diesem Verfahren wird jedoch die Stabilisierung des Wirkstoffs erreicht durch den Einsatz einer sehr spezifischen Gerüstbildner-Kombinationen bestehend aus einem Oligosaccharid, Zuckeralkohol und einem wasserlöslichem Polymer in spezifischen Mengenverhältnissen. Aus den offenbarten Mengenverhältnissen ergibt sich, dass auch hier lediglich solche gefriergetrockneten wirkstoffbeladenen Formkörper erhalten werden, in denen der Anteil der stabilisierenden Gerüstbildner den Anteil an Wirkstoff bei weitem übersteigt.

Sowohl für die kosmetische als auch für die pharmazeutische Anwendung von Einzelapplikations-Formkörpern, sind relativ große Formkörper einheitlicher Form und Größe bevorzugt, da diese im Gegensatz zu Pulvern oder kleinen Pellets, Mikroperlen und Granulaten durch den Endverbraucher einfacher zu handhaben sind, so dass das Bestreben dahin ausgerichtet ist, Formkörper einer solchen Größe bereitzustellen, die es erlauben pro Anwendung eine Dosierungsform zu benutzen. Außerdem vermitteln größere Formkörper, die z.B. farblich gestaltet werden können, auch einen stärkeren ästhetischen Eindruck.

Die Anwendung von Protein-Gerüstbildnern ist dabei in einigen Fällen nicht bevorzugt. So ziehen einige Endverbraucher zunehmend die Anwendung rein pflanzlicher Produkte insbesondere in der Kosmetik vor. Die Gründe hierfür resultieren dabei unter anderem aus grundsätzlichen, ethischen Erwägungen.

Die Aufarbeitung von Proteinen erfordert weiterhin in der Regel komplizierte Aufbereitungs- und Reinigungsschritte. Ferner sind die Eigenschaften der Protein-Gerüstbildner bei der äußeren Anwendung auf der Haut in ihrem Eigenschaftsspektrum in vielen Fällen zu beschränkt, da sie stets aus denselben Aminosäuren zusammengesetzt sind.

Um nun besonders schnell- und möglichst vollständig lösliche großformatige Formkörper mit einer sowohl in der Kosmetik als auch der pharmazeutischen Anwendung gewünschten hohen Wirkstoffbeladung zu erhalten, die insbesondere die stabile Bereitstellung von feuchtigkeitsempfindlichen, und insbesondere sogenannten sauren Wirkstoffen wie beispielsweise Ascorbinsäure (Vitamin C) und seinen Derivaten oder Acetylsalicylsäure und seinen Derivaten ermöglicht, sind somit aus den genannten Gründen solche Gerüstbildner, die nicht proteinogen Ursprungs sind, wie insbesondere pflanzliche polymere Gerüstbildner wie hochmolekulare Polysaccharide z. B. Alginate oder tierische Polyaminosaccharide wie Chitin oder dessen Derivate, insbesondere Chitosan, auch aufgrund ihres Lösungsverhaltens und ihrer hohen Gelbildungskapazität zur Herstellung solcher gefriergetrockneter Wirkstoff-Formkörper besonders geeignet.

So beschreibt beispielsweise die DE 10248314, auch als WO 2004/035023 veröffentlicht, regelmäßig geformte großformatige mit Wirkstoffen beladene Formkörper, die erhältlich sind durch ein Gefriertrocknungsverfahren einer in Formen gegossenen Wirkstoff-Gerüstbildner-Mischung. Die nach dem beschriebenen Verfahren erhältlichen Formkörper zeichnen sich durch eine gute mechanische Stabilität und eine hohe Auflösungsgeschwindigkeit aus. Die beschriebenen Formkörper sind erhältlich mit Mengen an pflanzlichem Gerüstbildner von mindestens 10 Gew.-% bezogen auf die Gesamtzusammensetzung. Als mögliche Wirkstoffgehalte werden zwar prinzipiell Mengen von 0-85 Gew.-% angegeben, wobei jedoch Wirkstoffbeladungen ≤ 50 Gew.-% als bevorzugt herausgestellt werden. Von den Beispielen gestützt sind lediglich Ausführungsformen bis maximal 25 Gew.-% Wirkstoff (Beispiel 4). Auch ergeben sich aus diesem Dokument keine Anhaltspunkte über einen spezifischen pH-Wert der dort offenbarten Formkörper wie insbesondere über einen pH-Wert < 7 oder gar ≤ 6 bzw. ≤ 4. Als mögliche Wirkstoffe werden zahlreiche Gruppen und Wirkstoffklassen aufgelistet. Eine spezielle Ausrichtung auf die Stabilisierung von instabilen Wirkstoffen, insbesondere solchen, die eine geringe Feuchtigkeitsstabilität aufweisen und in neutralem bis alkalischem Milieu eine hohe Dissoziationsneigung aufweisen (sogenannte saure Wirkstoffe), kann diesem Dokument nicht entnommen werden, ebenso wenig wie ein Hinweise auf eine Stabilisierung derartiger instabiler saurer Wirkstoffe durch eine spezifische pH-Wert-Einstellung. Die Auflösegeschwindigkeit der beschriebenen Formkörper liegt im Bereich von < 4 Minuten, diese ist prinzipiell abhängig vom jeweiligen Gehalt an Gerüstbildnern.

Grundsätzlich sind solche Zubereitungen mit besonders geringem Anteil an Gerüstbildnern schneller und vollständiger löslich als solche mit hohen Gerüstbildner-Anteilen.

Bei besonders großformatigen Formkörpern, besonders solchen auf Basis pflanzlicher Gerüstbildner, wie z.B. Alginaten, und deren Einsatz in sehr geringem Verhältnis zum vorhandenen Wirkstoff ergibt sich jedoch schnell das Problem, eine ausreichende mechanische Stabilität zu erhalten, um Formkörper mit regelmäßiger Ausgestaltung und mit schneller und vollständiger Löslichkeit, wie sie sowohl für die äußeren Anwendungen als auch für eine orale Applikation erforderlich sind, herstellen zu können.

Besonders die Einarbeitung und Stabilisierung von hohen Mengen solcher feuchtigkeitslabilen, sauren Wirkstoffe wie Ascorbinsäure (Vitamin C) und seiner Derivate oder Acetylsalicylsäure und seiner Derivate, die neben der hohen Feuchtigkeitsempfindlichkeit außerdem aufgrund der Dissoziationsneigung eine gefrierpunkterniedrigende Wirkung aufweisen, ist in gefriergetrockneten Zusammensetzungen wie solchen in der DE 10248314 offenbarten Gerüstbildner-haltigen gefriergetrockneten Formkörpern bisher nicht möglich. Durch die gefrierpunkterniedrigende Wirkung solcher dissoziierten Wirkstoffe bilden sich im gefrorenen Formkörper in der Regel große Eiskristalle mit einem hohen Anteil nicht einfrierbarem Wasser mit hohen Aktivstoffkonzentrationen bzw. Ionenkonzentrationen, die zu einem partiellen Strukturkollaps des gefriergetrockneten Endproduktes, zu einem sogenannten Antauen des Formkörpers führen, so dass die Herstellung mechanisch stabiler, einheitlicher und ansprechend aussehender Formkörper mit den beschriebenen Verfahren bisher nicht durchführbar ist.

Die DE 2017373 löst das Problem der Antauproblematik von hohen Mengen gefrierpunkterniedrigenden Vitamin C's bei der Herstellung mechanisch stabiler, schnell und vollständig löslicher, feuchtigkeitsstabiler, dosierbarer pharmazeutischer Einzelapplikationsformen, indem der mit ca. 30 Gew.-% bereits ungewöhnlich hohe Anteil an gefrierpunkterniedrigendem Vitamin C zusammen mit einer Menge von ca. 70 Gew.-% Gerüstbildner und proteinogenem Füllstoff Glycin vermischt wird, nachdem das Vitamin C zuvor mit einem synthetischen Blockcopolymer verschäumt worden ist.

Der Einsatz solcher synthetischer Blockcopolymere ist jedoch in kosmetischen und pharmazeutischen Zubereitungen unerwünscht, da prinzipiell die Verwendung von synthetischen Substanzen oder von Träger-, Hilfs- oder Zusatzstoffen ohne eigentliche pharmakologische Wirkung so gering wie möglich gehalten werden sollte, um mögliche toxikologische oder pharmakologische Nebeneffekte zu vermeiden. Darüber hinaus setzen, wie bereits ausgeführt, Zusätze an polymeren Trägersubstanzen prinzipiell mit zunehmendem Anteil die Löslichkeit der Zubereitungen herab, weswegen es wünschenswert ist, mit so geringen Mengen wie möglich zu arbeiten und dabei möglichst natürliche und toxikologisch unbedenkliche Polymer- oder Trägersubstanzen zu verwenden.

Die Aufgabe der vorliegenden Erfindung bestand somit darin, eine gefriergetrocknete Zusammensetzung zur Verfügung zu stellen, in der extrem hohe Mengen instabiler, insbesondere sogenannter saurer Wirkstoffe (Wirkstoffe mit einem pKs-Wert bei 25 °C von $\leq 7$), langfristig stabilisiert gehalten und bei der Anwendung schnell, effizient, spezifisch und hochaktiv freigesetzt und appliziert werden können. Des weiteren bestand die Aufgabe darin, für diese gefriergetrockneten Zusammensetzungen eine Möglichkeit zur Einarbeitung hoher Mengen solcher saurer Wirkstoffe zu finden, die prinzipiell eine gefrierpunkterniedrigende Wirkung haben und somit in solchen Mengen für die Herstellung gefriergetrockneter Zubereitungen bislang nicht einsetzbar waren. Darüber hinaus bestand die Aufgabe außerdem darin, diese stabilen Wirkstoff-Zusammensetzungen so auszugestalten, dass sie eine hohe mechanische Festigkeit und eine ausreichende Größe aufweisen, um insbesondere zur kosmetischen oder pharmazeutischen Applikation in Form von sogenannten singledosage-units oder Einzeldosisanwendung verwendet werden zu können. Dabei sollen die Zusammensetzungen gleichermaßen geeignet sein für die äußerliche Anwendung sowie für eine orale oder perorale Applikation.

Überraschend wurde gefunden, dass ausgehend von der o.g. DE 10248314 (WO 2004/035023) auch solche stabilen, großformatigen wirkstoffbeladenen Formkörper hergestellt werden können, die Wirkstoffmengen enthalten, die mit > 50 Gew.-% Wirkstoffgehalt und $\leq 15$ Gew.-% Gerüstbildneranteil wobei Proteine ausgenommen sind, wobei mindestens ein Gerüstbildner Chitosan ist, im äußersten Grenzbereich der DE 10248314 und insbesondere darüber hinaus liegen. Dabei können überraschend insbesondere instabile, saure Wirkstoffe, die aufgrund ihrer gefrierpunkterniedrigenden Wirkung bisher in derart großen Mengen nicht in gefriergetrockneten Zusammensetzungen der vorliegenden Art einarbeitbar waren, eingebracht werden, indem die Zubereitungen, in die die Wirkstoffe eingebracht werden, zuvor auf einen pH-Wert < 7, bevorzugt $\leq 6$, bevorzugter $\leq 5$, besonders bevorzugt $\leq 4$ eingestellt werden.

Aus der DE 102006038629 sind gefriergetrocknete Zusammensetzungen bekannt, die mindestens 10 Gew.% Trägermaterialien sowie bis zu 50 Gew.% Wirkstoffe in stabilisierter Form, bevorzugt in Form von Wirkstoffderivaten und/oder -precursorn enthalten. Dabei werden als Wirkstoffe solche aus der Gruppe der Vitamine, wie insbesondere Vitamin C (Ascorbinsäure) und Derivate als bevorzugt genannt. Darüber hinaus enthalten die Zusammensetzungen bis zu 50 Gew.% eines Agens zur Bildung des Wirkstoffes aus der stabilisierten Form bei Zutritt einer wässrigen Phase zur Zusammensetzung. Derartige Freisetzungsagentien werden bevorzugt aus der Gruppe der Enzyme gewählt. Aus der DE 102006038629 ergeben sich jedoch keine Anhaltspunkte über einen spezifischen pH-Wert der dort offenbarten Formkörper wie insbesondere über einen pH-Wert < 7 oder gar $\leq 6$ bzw. $\leq 4$. Lediglich im Zusammenhang mit den bevorzugt verwendeten Trägermaterialien aus der Gruppe der Alginate wird für diese ein bevorzugter pH-Wert von 6-8 genannt. Dieser dient jedoch ausschließlich zur Charakterisierung der eingesetzten Alginate und lässt keine Rückschlüsse auf einen pH-Wert der letztendlich erhältlichen erfindungsgemäßen Formkörper zu.

Die korrespondierende internationale Anmeldung WO 2008/020066 geht über den Offenbarungsgehalt der o.g. DE 102006038629 insofern hinaus, als hierin zusätzlich die Ausführungsbeispiele 1 bis 5 enthalten sind. Beispiele 1 bis 3 offenbaren dabei die Herstellung einer entsprechenden gefriergetrockneten Zusammensetzung, worin eine Einstellung des pH-Wertes auf 4-5 vorgenommen wird und worin die erhältlichen Zusammensetzungen ca. 16 Gew.% Trägermaterial und ca. 1,6 Gew.% stabilisierten Wirkstoff (Ascorbylglucosid) aufweisen. Außerdem wird den Zusammensetzungen ca. 82 Gew.% des Enzyms Glucoamylase in Form einer handelsüblichen Enzymlösung (Novozym 300 GL; 10-40 %) sowie ggf. weitere Hilfsstoffe zugesetzt. Die handelsübliche Enzymlösung Novozym 300 GL enthält 10 bis 40 % Glucoamylase, womit diese in der Zusammensetzung mit einem Gehalt von ca. 8 bis maximal 33 Gew. % enthalten sein kann.

Zwar können prinzipiell Enzyme auch eine Aktivität als Wirkstoff entfalten, im vorliegenden Fall werden Enzyme von der Definition der Wirkstoffe, die insbesondere in Form stabilisierter Derivate vorliegen, nicht umfasst. Die Glucoamylase wird den Zusammensetzungen lediglich als Freisetzungsagens zugesetzt, die unter Flüssigkeitszutritt den aktiven Wirkstoff Ascorbinsäure freisetzt. Darüberhinaus wird in jedem Fall, auch unter Berücksichtigung der Enzyme, durch die Beispiele 1-3 der WO 2008/020066 ein Wirkstoffgehalt ≥ 50 Gew. % nicht offenbart. Damit offenbaren die Dokumente DE 102006038629 und WO 2008/020066 keine gefriergetrockneten Zusammensetzungen mit einem Wirkstoffgehalt > 50 Gew. % und einem pH-Wert < 7 oder gar ≤ 6 bzw. ≤ 4.

Aus der FR 2 886 845 sind darüber hinaus trockene Zusammensetzungen bekannt, die Gerüstbildner wie Natriumalginate in einer Menge von 15-75 Gew.% sowie Ascorbinsäure mit einem Gehalt von 0,1-80 Gew.% enthalten können und die u.a. durch Lyophilisation erhalten werden können. Dieses Dokument gibt jedoch keine konkreten Hinweise auf einen spezifisch zu wählenden pH-Wert, zumal die Aufgabe der Stabilisierung des instabilen Wirkstoffs Ascorbinsäure von der FR 2886845 durch die Zugabe des Maleinsäure-Copolymers gelöst wird. Damit ergeben sich aus diesem Dokument auch keine Hinweise auf eine gefriergetrocknete Zusammensetzung mit einem Wirkstoffgehalt ≥ 50 Gew. % und einem spezifischen pH-Wert < 7 oder gar ≤ 6 bzw. ≤ 4. Ein konkreter pH-Wert (pH 6) ist lediglich im Zusammenhang mit dem Ausführungsbeispiel 1 angegeben, wobei die dort offenbarte Zusammensetzung keine besonders hohen Wirkstoffgehalte von ≥ 50 Gew. % offenbart. Damit kann auch der FR 2 886 845 kein gefriergetrockneter Formkörper mit einem Wirkstoffgehalt ≥ 50 Gew. % und einem spezifischen pH-Wert von < 7 oder gar ≤ 6 bzw. ≤ 4 entnommen werden.

EP 0888769 offenbart kosmetische Zusammensetzungen in lyophilisierter Form enthaltend 10 bis 30 Gew.-% Alginate und 70 bis 90 Gew.-% Wirkstoffe, wobei als Wirkstoffe Pflanzenextrakte, Algenextrakte, Mineralstoffe und Spurenelemente sowie marine Proteine wie insbesondere marine Kollagene bzw. marine Nukleotide genannt werden. Wirkstoffe aus der Gruppe der sauren Wirkstoffe werden nicht offenbart. Desweiteren ergeben sich auch aus der EP 0888769 keine Hinweise auf einen spezifischen pH-Wert der erfindungsgemäßen Zusammensetzungen, wie insbesondere auf einen pH-Wert von < 7 oder gar ≤ 6 bzw. ≤ 4.

Damit ist es überraschend gelungen, einen gefriergetrockneten, mechanisch stabilen, großformatigen Wirkstoff-Formkörper zu erhalten, der darüber hinaus aufgrund des erfindungsgemäß äußerst niedrigen Gerüstbildneranteils außerdem hinsichtlich seines Löslichkeitsverhaltens gegenüber bereits bekannten Systemen wie z. B. den in der DE 10248314 beschriebenen noch einmal deutlich verbessert werden konnte.

Des weiteren zeigte sich überraschend, dass durch die Auswahl von Chitosan als Gerüstbildner die Stabilität sowie das Auflösungsverhalten solcher gefriergetrockneten Wirkstoff-Formkörper mit sehr geringen Gerüstbildneranteilen noch weiter verbessert werden kann.

Dies ist maßgeblich auf die besonders gute Löslichkeit von Chitosan in sauren pH-Bereichen zurückzuführen.

Weder die DE 10248314 noch die DE 2017373 noch eines der anderen hier diskutierten Dokumente offenbart eine Rezeptur, mit derart niedrigen Gerüstbildneranteilen, insbesondere ausgewählt aus der Gruppe der Chitinderivate wie Chitosan oder kationisierter Stärke oder kationisierter Carboxymethylcellulose und entsprechend hohen Wirkstoffgehalten, insbesondere von sauren, gefrierpunkterniedrigenden Wirkstoffen die durch Einstellung des pH-Werts auf pH < 7, bevorzugt pH ≤ 6, bevorzugter pH ≤ 5, besonders bevorzugt pH ≤ 4 einarbeitbar werden, um zu porösen, gefriergetrockneten Formkörpern mit den entsprechenden gewünschten Eigenschaften hinsichtlich mechanischer Stabilität, Auflösungsverhalten und Größe für die Anwendung in der kosmetischen und pharmazeutischen Einzeldosis-Anwendung zu gelangen.

Die Erfindung stellt somit gefriergetrocknete Formkörper, bereit, die > 50 Gew. % eines oder mehrerer Wirkstoffe und ≤ 15 Gew. % eines oder mehrerer Gerüstbildner, wobei Proteine ausgenommen sind, wobei mindestens ein Gerüstbildner Chitosan ist, sowie gegebenenfalls einen oder mehrere Hilfsstoffe, jeweils bezogen auf die Gesamtzusammensetzung des gefriergetrockneten Formkörpers, enthält, und deren 1 Gew. %ige Lösung oder Suspension in Wasser bei 20 °C einen pH Wert < 7 aufweist.

Die Erfindung stellt darüber hinaus auch ein Verfahren zur Herstellung dieser gefriergetrockneten Formkörper sowie die Kombination solcher gefriergetrockneten Formkörper in Kit-of-parts Anordnungen zusammen mit wässrigen Lösungen sowie die Verwendung der gefriergetrockneten Formkörper und der Kit-of-parts Kombinationen zur pharmazeutischen und kosmetischen Anwendung bereit.

Unter einem Formkörper im Sinne der Erfindung versteht man einen regelmäßig geformten geometrischen Körper, z.B. insbesondere Kugeln, Quader, Pyramiden, Sterne aber auch natürlichen Formen nachgebildete Formkörper wie z.B. solche in der Form von Tieren, wie z.B. Meerestieren, wie z.B. Seesterne, Meeresfrüchte, wie Muscheln, etc. Pflanzen und Pflanzenteilen, wie Blätter etc. Nach dem weiter unten beschriebenen Verfahren zur Herstellung der erfindungsgemäß verwendeten Formkörper sind alle diese Formen zugänglich. Erfindungsgemäß bevorzugt sind einheitliche regelmäßige sphärische Formen wie insbesondere eine Kugelgeometrie, da diese sich insbesondere bei Inkorporierung großer Mengen saurer, gefrierpunkterniedrigender Wirkstoffe wie z. B. Ascorbinsäure (Vitamin C) und ihrer Derivate oder Acetylsalicylsäure und ihrer Derivate als besonders vorteilhaft aufgrund des besonders günstigen Oberfläche/Volumen Verhältnisses auszeichnet. Die Sublimationsstrecke durch das schon trockene Produkt ist nach allen Seiten hin symmetrisch und klein, was den Dampftransport durch das schon trockene Gut im Rahmen des Gefriertrocknungsprozesses erleichtert.

Erfindungsgemäß ist auch eine Mehrzahl der genannten Formkörper in einem Behältnis umfasst. Auch kann es sich um Mischungen von Formkörpern verschiedener Geometrien oder unterschiedlicher Größen handeln. Die Formkörper können einzeln abgepackt sein, bevorzugt liegt jedoch insbesondere in der kosmetischen Anwendung eine Mehrzahl der Formkörper nebeneinander in Kontakt in einem Behältnis vor. Die Volumina der verwendeten Formkörper sind aufgrund des Verfahrens ihrer Herstellung an sich nicht beschränkt. Zweckmäßig liegen die Volumina bevorzugt bei mindestens etwa 0,1 cm$^3$, bevorzugt 0,3 cm$^3$, bevorzugter mindestens etwa 0,5 cm$^3$, noch bevorzugter mindestens etwa 0,6 cm$^3$. Nach oben werden die verwendeten Volumina zweckmäßig auf bis zu etwa 6 cm$^3$, bevorzugt bis zu etwa 5 cm$^3$, bevorzugter bis zu etwa 4 cm$^3$ beschränkt. Die Größe der Formkörper wird unter anderem durch die gewünschte Applikationsform oder den Ort der äußeren Anwendung der Formkörper bestimmt. So ermöglicht bei der äußeren kosmetischen oder pharmazeutischen Verwendung die Applikation auf größeren Körperflächen oder den Haaren (z.B. der direkte Auftrag der angefeuchteten Formkörper auf dem Rücken etc., oder der Einsatz als Badezusatz) den Einsatz größerer Formkörper, wohingegen bei der Anwendung auf kleineren Körperpartien (z.B. Wange etc.) kleinere Formkörper bevorzugt sind.

Auch bei der Herstellung von Formkörpern zur oralen Applikation kann die Größe angepasst werden. So ist beispielsweise denkbar, die Formkörpergröße auf die entsprechende Zielgruppe abzustimmen, wobei denkbar ist, älteren Anwendern größere Formkörper mit besserer Handhabbarkeit anzubieten und z. B. jüngeren Anwendern oder Kindern solche, die in einem abgestimmten Verhältnis zu ihrer Körpergröße und der altersbedingt zu erwartenden Compliance bei der Anwendung stehen.

Der Durchmesser eines Formkörpers (maximaler Abstand zwischen zwei Punkten in einem Formkörper jedweder Geometrie) liegt zweckmäßig bei mindestens etwa 3 mm, bevorzugt mindestens etwa 5 mm, bevorzugter mindestens etwa 7 mm, noch bevorzugter mindestens etwa 8 mm bis hin zu zweckmäßig etwa 60 mm, bevorzugt etwa 50 mm, bevorzugter etwa 40 mm, noch bevorzugter etwa 30 mm.

Ein besonders bevorzugter Formkörper weist aus besagten Gründen eine im wesentlichen kugelförmige Geometrie auf, wobei der Durchmesser der Kugel zwischen 3 bis 30 mm, bevorzugt zwischen 5 und 20 mm, bevorzugter zwischen 7 und 15 mm, noch bevorzugter zwischen 8 und 14 mm liegt. Besonders bevorzugt sind Formkörper in Form einer Kugel mit einem Durchmesser von mindestens 6 mm.

Die erfindungsgemäßen gefriergetrockneten Formkörper enthalten mindestens einen oder mehrere Wirkstoffe, von mehr als 50 Gew.% bezogen auf die gefriergetrocknete Gesamtzusammensetzung. Wirkstoffe schließen insbesondere kosmetische oder therapeutische bzw. pharmazeutische, für die äußere Anwendung sowie für die orale oder perorale Applikation geeignete Wirkstoffe ein. Bevorzugt enthält der erfindungsgemäß verwendete Formkörper mindestens einen kosmetischen und/oder pharmazeutischen Wirkstoff. Dementsprechend handelt es sich bei den erfindungsgemäßen gefriergetrockneten Formkörpern bevorzugt um kosmetische oder therapeutische Mittel.

Kosmetische Formkörper bzw. unter Verwendung kosmetischer Wirkstoffe hergestellte Formkörper im Sinne der Erfindung sind im wesentlichen Mittel im Sinne des Lebens-mittel-, Bedarfsgegenstände-und Futtermittelgesetzbuches (LFGB), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäß verwendeten kosmetischen Formkörpern beispielsweise um Badepräparate, Hautwasch- und-reinigungsmittel, Hautpflegemittel, insbesondere Gesichtshautpflegemittel, Augenkosmetika, Lippenpflegemittel, Nagelpflegemittel, Fußpflegemittel, Haarpflegemittel, insbesondere Haarwaschmittel, Haarkonditionierungsmittel, Haarweichspüler etc., Lichtschutzmittel, Hautbräunungs- und -aufhellungsmittel, Depigmentierungsmittel, Deodorants, Antihydrotika, Haarentfernungsmittel, Insektenrepellents etc. oder derartige Mittel in Kombination.

Beispiele kosmetisch gegebenenfalls auch z.B. dermatologischer, therapeutisch wirksamer Verbindungen schließen ein: Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation wie z. B. Glycerin oder Harnstoff, Sonnenschutzmittel, Keratolytika, Radikalfänger für freie Radikale, Antiseborrhöika, Antischuppenmittel, antiseptische Wirkstoffe, Wirkstoffe zur Behandlung der Anzeichen der Hautalterung und/oder Mittel, die die Differenzierung und/oder Proliferation und/oder Pigmentierung der Haut modulieren, Vitamine wie Vitamin C (Ascorbinsäure) und ihre Derivate, wie beispielsweise Glycoside wie Ascorbylglucosid, oder Ester der Ascorbinsäure wie Natrium- oder Magnesium-Ascorbylphosphat oder Ascorbylpalmitat und -stearat L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetalsalze von L-Ascorbinsäuresulfatestern wie Natrium- und Kaliumsalze von L-Ascorbinsäuresulfatestern; Erdalkalimetalsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetalsalze wie Magnesium- und Calciumsalze von L-Ascorbinsäureestern; und trivalente Metallsalze wie Aluminiumsalze von L-Ascorbinsäureestern.

Wirkstoffe mit reizender Nebenwirkung, wie alpha-Hydroxysäuren, ß-Hydroxysäuren, alpha-Ketosäuren, ß-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, oder dekorative Stoffe wie Pigmente oder Farbstoffe und -partikel für Foundations, Make-up Formulierungen, und andere Mittel zur kosmetischen Verschönerung und farblichen Gestaltung von Augen-, Lippen-, Gesicht etc. sowie abrasive Mittel.

Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskrautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B.alkoholische Auszüge, Frischpflanzensaft, Preßsaft), Eleutherokokkus senticosus; Alkaloide: Rauwolfia (z.B. Prajmalin),Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte.

Bevorzugte kosmetische und pharmazeutische Wirkstoffe sind solche, die eine hohe Instabilität gegen Abbau oder Zersetzung wie insbesondere bedingt durch Feuchtigkeitszufuhr aufweisen und die darüber hinaus in wässriger Lösung einen sauren pH-Wert aufgrund saurer Gruppen im Wirkstoff erzeugen, sogenannte saure Wirkstoffe. Ein besonders bevorzugter insbesondere in der Kosmetik verbreiteter Wirkstoff aus der Gruppe dieser instabilen, sauren und gefrierpunkterniedrigenden Wirkstoffe ist die Ascorbinsäure (Vitamin C) und ihre Derivate oder auch Vitamin A und Derivate davon.

Im Unterschied zu den vorstehend beschriebenen im wesentlichen in der Kosmetik verwendeten Formkörpern handelt es sich bei den therapeutisch verwendeten Formkörpern (Arzneimittel) um solche, die mindestens einen pharmazeutischen bzw. therapeutischen insbesondere auch dermatologischen Wirkstoff enthalten und die im Sinne des Arzneimittelgesetzes unter anderem dazu bestimmt sind, Krankheiten, Leiden, Körperschäden oder krankhafte Beschwerden zu heilen, zu lindern oder zu verhüten. Solche Mittel bzw. Wirkstoffe sind sowohl für die äußere Anwendung als auch für die orale oder perorale Applikation bestimmt.

Bei Wirkstoffen für die äußere Anwendung handelt es sich insbesondere um hautaktive aber auch um transdermale Wirkstoffe. Sie schließen beispielsweise ein: Mittel zur Behandlung von Hautkrankheiten, äußerlich anwendbare Analgetika, z. B. Dextropropoxyphen, Pentazocin, Pethidin, Buprenorphin; Antirheumatika/Antiphlogistika (NSAR), z. B. Indometacin, Diclofenac, Naproxen, Ketoprofen, Ibuprofen, Flurbiprofen, Salicylsäure und-derivate wie Acetylsalicylsäure, Oxicame; Steroidhormone, z. B. Betamethason, Dexamethason, Methylprednisolon, Ethinylestradiol, Medroergotamin, Dihydroergotoxin; Gichtmittel, z. B. Benzbromaron, Allopurinol; Dermatika Externa, einschließlich antibakterielle Mittel wie z.B. Silbersalze oder kolloidales Silber, Antimykotika, antivirale Wirkstoffe, entzündungshemmende Wirkstoffe, juckreizstillenden Wirkstoffe, anästhesierende Wirkstoffe, z. B. Benzocain, Corticoide, Aknemittel, antiparasitäre Wirkstoffe; äußerlich anwendbare Hormone; Venentherapeutika; Immunsuppresiva etc. alle für die äußerliche Anwendung.

Bevorzugte therapeutische Mittel für die äußere Anwendung sind Analgetika, z.B. Immunsuppressiva, Hormone, Mittel zur Behandlung von Hautkrankheiten, wie der Neurodermitis, der atopischen Dermatitis etc., und Anti-Herpesmittel.

Therapeutische Wirkstoffe für die orale oder perorale Applikation können aus gewählt sein aus der Gruppe der Antihistaminika, der Antibiotika, der Peptidarzneistoffe, Antimykotika, Bronchialtherapeutika wie Antiasthmatika, Antitussiva, Mucolytica etc., Antidiabetica wie z. B. Glibenclamid, Hormone, Steroidhormone wie Dexamethason, Herzglycoside wie Digitoxin, Herz- und Kreislauftherapeutika wie z. B. Beta-Blocker, Antiarrhythmika, Antihypertonika, Calcium-Antagonisten etc., Psychopharmaka und Antidepressiva wie z. B. trizyklische Antidepressiva (NSMRI), Serotonin-Wiederaufnahme-Hemmer (SSRI), Noradrenalin-Wiederaufnahme-Hemmer (NRI), Serotonin-Noradrenalin-Wiederaufnahme-Hemmer (SNRI), Monoaminooxidase-Hemmer (MAO-Hemmer) etc., Neuroleptika, Antikonvulsiva oder Antiepileptika, Hypnotika, Sedativa, Anästhetika, Magen-, Darmtherapeutika, Lipidsenker, Analgetika wie z. B. Antimigränemittel, Paracetamol, Salicylsäure und -derivate wie Acetylsalicylsäure, Diclophenac, Ibuprofen, Ketoprofen, Naproxen etc. , Antiphlogistika, Vasodilatatoren, Diuretica, Gichtmittel, Zytostatika, Muskelrelaxantien, Pflanzenextrakte, Provitamine wie z. B. Beta-Carotin, Vitamine wie z. B. Vitamin C, A, B, E etc., Kieselerde, Mineralstoffe und Spurenelemente wie z. B. Kalium, Magnesium, Calcium, Selen, Iod etc., Diätsupplemente und Nahrungsergänzungsmittel usw. alle für die orale oder perorale Applikation.

Ein besonders bevorzugter pharmazeutischer Wirkstoff, der sowohl für die äußerliche als auch die orale oder perorale Applikation verwendet wird und ausgewählt ist aus der Gruppe der instabilen, sauren und gefrierpunkterniedrigenden Substanzen ist die Salicylsäure- und ihre Derivate wie Acetylsalicylsäure (ASS). Weitere bevorzugte instabile, saure und gefrierpunkterniedrigende therapeutische Wirkstoffe sind Clofibrinsäure, Ibuprofen, Gemfibrozil, Fenoprofen, Naproxen, Ketoprofen, Indomethacin, Bezafibrat, Tolfenaminsäure, Diclofenac, Meclofenaminsäure, Paracetamol, Acitretin, Acrivastin, Azelainsäure, Cromolyn, Ethacrynsäure, Furosemid, Penicillin und Derivate davon, Risedronsäure und Derivate davon, Liponsäure, und Ursodiol.

Die erfindungsgemäßen gefriergetrockneten Formkörper zeichnen sich aus durch einen Wirkstoffanteil > 50 Gew. %, bevorzugt ≥ 75 Gew. %, bevorzugter ≥ 90 Gew. %, noch bevorzugter ≥ 95 Gew. % jeweils bezogen auf die Gesamtzusammensetzung des gefriergetrockneten Formkörpers. Besonders bevorzugt ist ein Wirkstoffgehalt > 50 Gew.%.

Dabei handelt es sich insbesondere um solche Wirkstoffe, die ausgewählt sind aus der Gruppe der sauren Wirkstoffe, also um Wirkstoffe, die in wässriger Lösung einen sauren pH-Wert aufgrund saurer Gruppen im Wirkstoff erzeugen. Bei solchen sauren Wirkstoffen handelt es sich speziell um Wirkstoffe, deren 1 gewichtsprozentige Lösung oder Suspension in Wasser bei 20 °C einen pH Wert < 7 aufweist, bzw. um solche Wirkstoffe, die einen pKs-Wert bei 25 °C von ≤ 7 aufweisen.

Der pKs-Wert bezeichnet dabei den negativen dekadischen Logarithmus der Säurekonstante Ks. Die Säurekonstante Ks ist eine Stoffkonstante und gibt Aufschluss darüber, in welchem Maß ein Stoff (HA) in einer Gleichgewichtsreaktion mit Wasser unter Protolyse reagiert:

$$HA + H_2O \rightleftarrows H_3O^+ + A^-.$$

Dabei steht HA für eine Brønsted-Säure (nach Brønsted), die ein Proton $H^+$ an ein Lösungsmittel wie Wasser abgeben kann, wobei ein Anion $A^-$ zurückbleibt. Allgemeiner gilt die Brønsted'sche Definition auch für nichtwässrige Systeme, hier gilt für ein beliebiges, protonierbares Lösungsmittel Y:

$$HA + Y \rightleftarrows HY^+ + A^-.$$

Die Säurekonstante Ks bezeichnet dabei die Gleichgewichtskonstante dieser Reaktion und ist damit ein Maß für die Stärke einer Säure. Je stärker die Säure, desto mehr ist die Reaktion auf die rechte Seite verschoben. Somit ergibt sich, daß je kleiner der pKs-Wert, desto stärker die Säure.

Die Bestimmung des pKs-Wertes erfolgt über pH-Messung in einer sogenannten Halbtitration. Dabei wird eine Lösung der Säure bekannter Konzentration vorgelegt und der pH-Wert z.B. mittels pH-Mess-Sonde gemessen. Daraufhin wird die Säure teilweise mit einer Maßlösung einer Base der gleichen Wertigkeit der vorgelegten Säure neutralisiert. Hierbei wird exakt die Hälfte der Stoffmenge der Säure aus der Vorlage zugesetzt. Nun wird wiederum der pH-Wert bestimmt. Es gilt:

$$pK_S = -lgK_S = -lg\frac{c[H^+] \cdot c[A^-]}{c[HA]}$$

Da nach Zugabe der halben Stoffmenge gilt, dass c[A⁻] = c[HA], gilt für den sogenannten Halbtitrationspunkt, dass pKs = pH.

Derartige saure Wirkstoffe verfügen über eine pH-Wert abhängige hohe Dissoziationsneigung, wodurch in neutralen bis alkalischen pH-Wertebereichen der Wirkstoff in dissoziierter Form und somit in einer hohen Ionenkonzentration vorliegt. Eine derartig erhöhte Ionenkonzentration führt dann zu einer gefrierpunkterniedrigenden Wirkung mit den vorstehend beschriebenen nachteiligen Auswirkungen im Gefriertrocknungsprozess, weshalb solche sauren Wirkstoffe durch Gefriertrocknungsverfahren bisher nicht oder nur in sehr geringen Konzentrationen bzw. unter sehr hohem Kostenaufwand durch eine Prozessführung bei niedrigsten Temperaturen mit sehr langen Trocknungszeiten in zufriedenstellendem Maße in stabile gefriergetrocknete Formen überführt werden konnten.

In einer bevorzugten Ausführungsform enthalten die erfindungsgemäßen gefriergetrockneten Formkörper bezogen auf die Gesamtzusammensetzung des gefriergetrockneten Formkörpers > 50 Gew. % eines Wirkstoffs aus der Gruppe der Ascorbinsäure und ihrer Derivate.

In einer weiteren bevorzugten Ausführungsform enthalten die erfindungsgemäßen gefriergetrockneten Formkörper bezogen auf die Gesamtzusammensetzung des gefriergetrockneten Formkörpers > 50 Gew.% eines Wirkstoffs aus der Gruppe der Salicylsäure und deren Derivate, bevorzugt aus der Gruppe der Acetylsalicylsäure und ihrer Derivate.

Somit sind generell mit dem erfindungsgemäßen Verfahren und mit der erfindungsgemäßen Zusammensetzung insbesondere solche Wirkstoffe der Gefriertrocknung zuführbar, welche aufgrund ihrer pH-Wert abhängigen Dissoziationsneigung besonders in hohen Konzentrationen den Gefrierpunkt von Wasser herabsetzen und somit gefrierpunkterniedrigend wirken.

Als gefrierpunkterniedrigend (oder auch schmelzpunkterniedrigend) bezeichnet man das Phänomen, dass der Schmelzpunkt von Lösungen niedriger liegt als der der reinen Flüssigkeiten.

Die Gefrierpunktserniedrigung ΔT ist für verdünnte Lösungen proportional zur Molalität n des gelösten Stoffes:

$$\Delta T = E_n \cdot n$$

Dabei senkt sich der Gefrierpunkt pro Mol gelöstem Stoff pro Kilogramm Lösungsmittel um einen lösungsmittelspezifischen Wert. Diesen Wert nennt man die kryoskopische Konstante $E_n$, die nur vom Lösungsmittel und nicht vom gelösten Stoff abhängt (bei Wasser ist dieser Wert 1,86 (K·kg)/mol). Sie lässt sich aus dem Raoultschen Gesetz und der Clausius-Clapeyronschen Gleichung ableiten zu

$$E_n = R\frac{T_g^2}{L_S},$$

wobei

R die allg. Gaskonstante = 8,314472 J/(mol·K),
$T_g$ der Gefrierpunkt des Lösungsmittels in K und
$L_S$ die spezifische Schmelzwärme des Lösungsmittels in J/kg ist. Diese Beziehung gilt nur für stark verdünnte Lösungen (Konzentrationen <0,1 mol/L), bei höher konzentrierten Lösungen ist die Aktivität und nicht die Konzentration der Inhaltsstoffe und des Wassers zu beachten.

Es ist dabei zu beachten, dass Salze, Säuren und Basen (Elektrolyte) in wässriger Lösung dissoziieren. Infolgedessen ist durch diese elektrolytische Dissoziation die gefundene Gefrierpunktserniedrigung höher als die molare Konzentration erwarten lässt.

Speziell Wirkstoffe, die eine elektrolytische Dissoziation aufgrund ihrer sauren Wirkgruppen wie z. B. Ascorbinsäure (Vitamin C) und ihre Derivate oder Salicylsäure und ihre Derivate wie Acetylsalicylsäure (ASS) zeigen, können durch das erfindungsgemäße Verfahren, dass die Einstellung des pH-Wertes der Wirkstoffzusammensetzung auf einen Wert < 7 umfasst, auch in hohen Wirkstoffkonzentrationen einer Stabilisierung gegen Feuchtigkeit in schnell-löslichen großformatigen, mechanisch stabilen gefriergetrockneten Formkörpern zugänglich gemacht werden. Durch den gewählten pH-Bereich verschiebt sich das Säure/Base-Gleichgewicht auf die Seite der Säure, so dass keine dissoziierten Ionen vorliegen und die Wirksubstanzen elektrisch neutral vorliegen. Dadurch verringert sich die Tendenz zur Gefrierpunktserniedrigung und der Antauproblematik von Wirkstoffen mit elektrolytischer Dissoziationsneigung wird entgegengewirkt und hoch konzentrierte Wirkstoffzusammensetzungen können ohne Verluste in der Qualität und Stabilität der Formkörper gefriergetrocknet werden. Darüber hinaus ist die Bereitstellung von Wirkstoffzusammensetzungen, in denen die sauren Wirkstoffe in undissoziierter Form vorliegen, auch insofern bevorzugt, als undissoziierte Wirkstoffe wie beispielsweise undissoziierte Ascorbinsäure per se schon eine bessere chemische Stabilität aufweisen und darüber hinaus auch eine bessere Bioverfügbarkeit bei der Applikation aufweisen.

Eine gefriergetrocknete Formkörper-Zusammensetzung mit einem sauren pH-Wert ist darüber hinaus jedoch auch insofern vorteilhaft, da derartige saure Zusammensetzungen in der dermalen und topischen Anwendung eine verbesserte Hautpenetration der in diesem pH-Wertebereich ungeladenen Wirkstoffe ermöglichen.

Außerdem weisen saure gefriergetrocknete Formkörper, wie bereits ausgeführt, eine geringere Hygroskopie-Neigung auf, da die protonierten Formen der sauren Wirkstoffe weniger hygroskopisch wirken, als die unprotonierten, dissoziierten, in ionischer Form vorliegenden Wirkstoffe. Als ein Beispiel für einen sauren Wirkstoff in unprotonierter Form mit erhöhter Hygroskopie-Neigung wäre etwa Natriumascorbat zu nennen.

Der erfindungsgemäße gefriergetrocknete Formkörper weist somit in einer 1 gewichtsprozentigen Lösung oder Suspension in Wasser bei 20 °C einen pH Wert von < 7, bevorzugt $\leq$ pH 6,0, bevorzugter $\leq$ pH 5,0, besonders bevorzugt $\leq$ pH 4,0 auf. Besonders bevorzugt sind pH-Werte < 6 bzw. < 4.

Die erfindungsgemäßen gefriergetrockneten Formkörper enthalten außerdem mindestens einen Gerüstbildner, wobei Proteine ausgenommen wobei mindestens ein Gerüstbildner Chitosan ist, sind in einer Menge von $\leq$ 15 Gew.-% bezogen auf die Gesamtzusammensetzung. Bei dem Gerüstbildner wobei mindestens ein Gerüstbildner Chitosan ist, handelt es sich im allgemeinen um sogenannte Hydrokolloide, d.h. (teilweise) wasserlösliche, natürliche oder synthetische Polymere, die in wässrigen Systemen Gele bzw. viskose Lösungen bilden. Die Gerüstbildner werden zweckmäßig ausgewählt aus Polysacchariden, Mucopolysacchariden, tierischen Polyaminosaccharide wie Chitin oder dessen Derivaten, insbesondere Chitosan oder aus den Glucosaminoglykanen sowie den synthetischen Polymeren wie insbesondere aus der Gruppe der kationisch modifizierten Stärke oder der kationisch modifizierten Carboxymethylcellulose. Bevorzugt wird der Gerüstbildner aus der Gruppe der Polysaccharide ausgewählt. Polysaccharide schließen beispielsweise Homoglykane oder Heteroglykane ein, wie zum Beispiel Alginate, besonders Natriumalginat, Carrageen (hierin ggf. auch unter der gleichbedeutenden englischen Bezeichnung "Carragenan" geführt), Pektine, Tragant, Guar-Gummi, Johannisbrotkernmehl, Agar-Agar, Gummi-Arabikum, Pullulan, Trahalose, Xanthan, natürliche und modifizierte, wie z.B. kationisch modifizierte Stärken, Dextrane, Dextrin, Maltodextrine, Glucane, wie ß-1,3-Glucan, ß-1,4-Glucan, wie Cellulose, Mucopolysaccharide, wie Hyaluronsäure etc., sowie tierische Polyaminosaccharide wie Chitin oder dessen Derivate wie insbesonders Chitosan. Synthetische Polymere schließen beispielsweise ein: Celluloseether, Polyvinylalkohol, Polyvinylpyrrolidon, synthetische Cellulosederivate, wie Methylcellulose, Carboxycellulose, Carboxymethylcellulose, kationisch modifizierte Carboxymethylcellulose, Celluloseester, Cellulosesether wie Hydroxypropylcellulose, Polyacrylsäure, Polymethacrylsäure, Poly(methylmethacrylat) (PMMA), Polymethacrylat (PMA), Polyethylenglykole etc. Es können auch Mischungen mehrerer Gerüstbildner verwendet werden.

In einer bevorzugten Ausführungsform enthalten die erfindungsgemäßen gefriergetrockneten Formkörper mindestens ein Gerüstbildner Chitosan ist.

Im allgemeinen fallen unter den Begriff der kationischen Polymere bzw. Gerüstbildner insbesondere modifizierte Chitinderivate wie insbesondere Chitosan,

In einer besonders bevorzugten Ausführungsform ist der Gerüstbildner aus der Gruppe der kationischen Gerüstbildner Chitosan

Weitere erfindungsgemäß besonders bevorzugte Gerüstbildner sind Hyaluronsäure, und Alginate, wie insbesondere Natrium-Alginat.

Der Einsatz geringer Mengen von gerüstbildenden Substanzen in den erfindungsgemäßen Wirkstoff-Formkörpern gegenüber beispielsweise reinen, getrockneten, Zusatzstoff-freien Wirkstoffen ist zum einen notwendig, um den Wirkstoff unmittelbar in einer geeigneten Darreichungsform bereitstellen zu können, was insbesondere in der äußerlichen Anwendung hinsichtlich Applizierbarkeit und Handhabbarkeit eine Rolle spielt. Dabei ist insbesondere bei solchen äußerlich anzuwendenden Wirkstoff-Formkörpern der Gerüstbildner-Anteil für die Texturbildung des rehydratisierten bzw. aufgelösten Wirkstoff-Formkörpers wichtig, damit der aufgelöste Wirkstoff auf die Haut oder das Haar gut aufgetragen werden kann. Reine wässrige Wirkstoff-Lösungen hingegen würden "weglaufen" und sind aus naheliegenden Gründen aufgrund der fehlenden Viskosität für die äußerliche Verwendung nicht praktikabel.

Zum Anderen tragen die Gerüstbildner-Anteile bereits in den erfindungsgemäßen geringen Mengen entscheidend zur Stabilität der Wirkstoff-Formkörper bei. Durch diese bereits geringen Mengen gelingt die Überführung der Wirkstoffe in eine regelmäßige, einheitliche, geometrische Form mit einer ausreichenden mechanischen Stabilität bei der Verpackung, Lagerung, Handhabung und Anwendung. Insbesondere wurde überraschend gefunden, dass die erfindungsgemäßen Gerüstbildner-Anteile bereits ausreichen, um nicht staubende und gegen mechanischen Abrieb hinreichend geschützte Wirkstoff-Formkörper bereitstellen zu können.

Die erfindungsgemäß bevorzugt als Gerüstbildner verwendeten Polysaccharide weisen zweckmäßig durchschnittliche Molmassen von etwa $10^3$ bis zu etwa $10^8$, bevorzugt etwa $10^4$ bis $10^7$ auf.

Die erfindungsgemäßen gefriergetrockneten Formkörper zeichnen sich dadurch aus, dass sie einen Gerüstbildneranteil wobei Proteine ausgenommen sind, wobei mindestens ein Gerüstbildner Chitosan ist, $\leq$ 15 Gew. %, bevorzugt $\leq$ 10 Gew. %, bevorzugter $\leq$ 5 Gew. %, jeweils bezogen auf die Gesamtzusammensetzung des gefriergetrockneten Formkörpers enthalten.

Die wobei Proteine ausgenommen sind, wobei mindestens ein Gerüstbildner Chitosan ist, sind haut- und schleimhautverträglich und weisen weder bei der äußerlichen noch bei der oralen oder peroralen Applikation toxikologisches Potential, Irritationswirkungen oder andere Unverträglichkeitsreaktionen hervor. Sie sind pharmakologisch völlig unbedenklich und somit optimal geeignet als Trägermaterial für die erfindungsgemäßen kosmetischen und pharmazeutischen äußerlichen und oralen bzw. peroralen Verwendungen.

Klarstellend soll noch erwähnt werden, dass die Formulierung "Gerüstbilder, wobei Proteine ausgenommen sind," im Sinne der Erfindung nicht die Anwesenheit von Wirkstoffen auf Proteinbasis, wie Enzyme, Hormone etc. ausschließt. Auch die Gerüstbildner, insbesondere die Polysaccharide können gewisse therapeutische Wirkungen aufweisen. So wirkt der bevorzugt verwendete Gerüstbildner (Natrium-)Alginat in gewissen Ausmaß antiviral und Hyaluronsäure wird eine gewisse Wirkung in der Re-Epithelisierung und als Antioxidans und Feuchtigkeitsspender in der Hautpflege nachgesagt, sie sind jedoch keine Wirkstoffe im Sinne der Erfindung.

Die erfindungsgemäß verwendeten Formkörper enthalten weiterhin gegebenenfalls einen oder mehrere Hilfsstoffe. Hilfsstoffe schließen ein: pH-Einstellungsmittel, wie Pufferstoffe, anorganische und organische Säuren oder Basen, Fettsubstanzen, wie Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Neutralöl, Fettsäureester, Ester von Fettalkoholen wie Triglyceride und Wachse mit einem der

Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle (sogenannte kosmetische Öle), wie beispielsweise in der CTFA-Abhandlung, Cosmetic Ingredient Handbook, 1. Auflg., 1988, The Cosmetic, Toiletry and Fragrance Association, Inc., Washington, erwähnt, oberflächenaktive Mittel neben den oben erwähnten Waschtensiden, wie Dispergiermittel, Emulgatoren etc., Füllstoffe, Stabilisatoren, Cosolventien, pharmazeutisch und kosmetisch gebräuchliche oder sonstige Farbstoffe und Pigmente, insbesondere solche, die primär zur farblichen Gestaltung der Formkörper eingesetzt werden und nicht zur Applikation und farblichen Gestaltung am menschlichen Körper, wie solche Pigmente und Farbstoffe wie die unter der Gruppe der Wirkstoffe aufgeführten dekorativen Farbstoffe, Konservierungsmittel, Weichmacher, Schmiermittel bzw. Gleitmittel, etc.

Ein besonders bevorzugter Hilfsstoff ist Salzsäure zur Einstellung des gewünschten pH-Werts von < 7, bevorzugt $\leq$ 6, bevorzugter $\leq$ 5, besonders bevorzugt < 4.

Außerdem bevorzugt sind Hilfsstoffe aus der Gruppe der Fette und Öle, insbesondere aus der vorstehend genannten Gruppe der kosmetischen Öle, wie insbesondere Jojobaöl, Squalan oder Capryl/Capronsäure-Triglyceride (Neutralöl). Weitere erfindungsgemäß bevorzugte Hilfsstoffe sind pH-regulierende Puffersubstanzen und inerte Füllstoffe.

Generell schließt die Einordnung der vorstehend erwähnten Stoffe in die Kategorie der Hilfsstoffe im Rahmen der vorliegenden Erfindung nicht aus, dass diese Hilfsstoffe auch gewisse kosmetische und/oder therapeutische Wirkungen entfalten können, was in besonderem Maß für die genannten bevorzugt eingesetzten kosmetischen Öle gilt.

Hilfsstoffe können den erfindungsgemäßen gefriergetrockneten Formkörpern in Mengen bis zu 25 Gew.-% bezogen auf die Gesamtzusammensetzung hinzugefügt werden.

Die erfindungsgemäßen Formkörper dienen der äußeren kosmetischen und pharmazeutischen sowie der oralen oder peroralen Anwendung bei Menschen oder Tieren. Die äußere Anwendung erfolgt dabei so, dass der erfindungsgemäße Formkörper mit Wasser bzw. einer wässrigen Lösung, die einen oder mehrere Wirkstoffe und/oder einen oder mehrere Hilfsstoffe enthält, angefeuchtet oder gelöst wird. Je nach Flüssigkeitsmenge und Löslichkeit des Formkörpermaterials das verwendet wird, kann der Formkörper vollständig unter Bildung einer Lösung gelöst werden oder unter Bildung eines Gels zerfallen.

Bevorzugt werden dabei wässrige Lösungen verwendet, die außerdem feuchthaltende Alkohole wie z.B. Glycerin, Butylen- oder Pentylenglykol enthalten, sowie solche, die niedrigviskos sind (eine Viskosität < 50 mPas) und keine oder lediglich geringe Ölanteile (< 10 Gew.-% bezogen auf die Gesamtzusammensetzung der wässrigen Lösung) enthalten. Weiterhin bevorzugt sind solche Aktivatorlösungen, die frei sind von Erdalkaliionen wie insbesondere Calcium- und/oder Magnesiumionen (weniger als 1 Gew-% Calcium- und/oder Magnesiumionen bezogen auf die Gesamtzusammensetzung der wässrigen Lösung enthalten), sowie solche mit einem pH-Wert zwischen etwa pH 4,0 bis 8,0.

Erfindungsgemäß ist in der äußeren Anwendung ebenfalls enthalten, die Lösung des erfindungsgemäßen Formkörpers in einer für eine Badeanwendung geeigneten Wassermenge. Bevorzugt erfolgt die Anwendung jedoch so, dass die Formkörper mit einer kleinen Menge von etwa 0,5 bis 5,0 ml Wasser oder einer Wirk- und/oder Hilfsstofflösung unter Bildung einer Lösung oder eines Gels direkt auf der Haut oder im Haar oder in einem geeigneten Gefäß befeuchtet werden und dort innerhalb von ≤ 30 Sekunden vollständig zerfällt. Bevorzugt wird der gefriergetrocknete Formkörper dabei rückstandslos aufgelöst. Dabei erfolgt die Auflösung vorzugsweise unter leichter mechanischer Einwirkung z. B. durch Verrühren, Verreiben, Zerdrücken bzw. Massieren.

Die vorliegende Erfindung betrifft auch eine Kombination, enthaltend mindestens einen der erfindungsgemäß verwendeten Formkörper sowie mindestens eine wässrige Lösung, die einen oder mehrere Wirkstoffe und/oder mindestens einen oder mehrere Hilfsstoffe enthält (eine sogenannte Aktivatorlösung), in einer zusammengehörenden, räumlichen Anordnung (Anwendungspaket, Set, Kit-of-Parts etc.). Bei der Wirkstofflösung kann es sich z.B. um Lösungen von leicht-flüchtigen Wirk- und/oder Hilfsstoffen handeln, die aufgrund des Herstellverfahrens durch die Gefriertrocknung nicht in den Formkörper eingebracht werden sollen bzw. können, wie z.B. gewisse Anteile ätherischer Öle, Parfums etc. Auch können solche Wirk- und/oder Hilfsstoffe enthalten sein, die eine befeuchtende Wirkung erzielen, die insbesondere bei der äußerlichen Anwendung auf der Haut erwünscht und bevorzugt ist, und die aufgrund dieser befeuchtenden Wirkung oder aufgrund von Hygroskopie-Neigungen nicht in den erfindungsgemäßen gefriergetrockneten Formkörper eingearbeitet werden können, da dadurch die Stabilität der feuchtigkeitslabilen Wirkstoffe nicht mehr aufrechterhalten werden kann.

Die Ausgestaltung solcher Kit-of-parts Kombinationen aus erfindungsgemäßem Formkörper einerseits und Wirkstofflösung andererseits kann dabei vorsehen, dass die beiden Bestandteile separat aus der Kit-of-parts Anordnung entnommen werden und außerhalb davon für die weitere Verwendung zusammengeführt und aufgelöst werden. Es ist jedoch auch denkbar, dass eine Zusammenführung der beiden Komponenten innerhalb der Kit-of-parts-Verpackung selbst erfolgt und die aufgelöste Zusammensetzung sodann aus dieser direkt der weiteren kosmetischen oder pharmazeutischen äußerlichen, oralen und/oder peroralen Verwendung zugeführt wird. Dies kann bevorzugt direkt durch den Endverbraucher durchgeführt werden.

Die erfindungsgemäßen Formkörper enthalten > 50 Gew.-%, bevorzugt 75 Gew.-% oder mehr, bevorzugter 90 Gew.-% oder mehr, noch bevorzugter 95 oder mehr Gew.-% eines oder mehrerer Wirkstoffe. Besonders bevorzugt sind feuchtigkeitslabile, saure Wirkstoffe, insbesondere solche mit elektrolytischer Dissoziationsneigung und somit gefrierpunktserniedrigender Wirkung wie insbesondere Ascorbinsäure (Vitamin C) und ihre Derivate und/oder Salicylsäure und ihre Derivate wie Acetylsalicylsäure (ASS).

Der Gehalt der Wirkstoffe in der trockenen Gesamtzusammensetzung kann bestimmt werden durch geeignete anerkannte Analysemethoden wie z. B. nach DIN, Pharmacopeia, Amtliche Sammlung von Untersuchungsverfahren (ASU) etc. Die Wahl der geeigneten Methode hängt dabei natürlich von der Art des Wirkstoffes ab. Insbesondere die besonders bevorzugten Wirkstoffe wie Ascorbinsäure (Vitamin C) und ihre Derivate und/oder Salicylsäure und ihre Derivate wie Acetylsalicylsäure (ASS) können mittels Hochleistungsflüssigkeitschromatographie-Methoden (HPLC) untersucht werden. HPLC-Methoden zur quantitativen Vitamin C und Acetylsalicylsäurebestimmung können, ggf. mit Anpassungen der Probenaufbereitung, z.B. aus den offiziellen Monografien "Aspirin Tablets" und "Ascorbic acid Injections" aus der USP 31, NF 26 Volume 2, 2008 entnommen werden.

Je nach vorhandener Menge und Art der vorhandenen Wirkstoffe und/oder gegebenenfalls zusätzlichen Hilfsstoffe enthält der erfindungsgemäße Formkörper maximal etwa 15 Gew.-% des Gerüstbildners, bezogen auf das Gesamtgewicht des gefriergetrockneten Formkörpers, bevorzugt maximal bzw. weniger als 10, bevorzugter maximal 7, noch bevorzugter weniger als 5 Gew.-% des Gerüstbildners, jeweils bezogen auf die Gesamtzusammensetzung des gefriergetrockneten Formkörpers, wobei Proteine ausgenommen sind, wobei mindestens ein Gerüstbildner Chitosan ist,

Der integrale Anteil an Gerüstbildnern in der trockenen Gesamtzusammensetzung kann dabei durch Hydrolyse der vorliegenden Polymerketten mit anschließendem quantitativen, chromatografischem Nachweis der einzelnen Monomerbausteine bestimmt werden. Ist diese Methode durch eine spezielle Kombination unterschiedlicher Gerüstbildner und spezieller Wirk- wie Hilfsstoffe nicht einsetzbar, kann der quantitative Polymeranteil mathematisch über die Differenz zwischen Gesamtgewicht und quantitativ bestimmbaren Hilfs- und Wirkstoffen und Wasser bestimmt werden. Die quantitativen Methoden zur Bestimmung der einzelnen Rezepturkomponenten entlehnt man aus den schon vorher genannten offiziellen Methodensammlungen.

Die Formkörper können bis zu etwa 25 Gew.-% eines oder mehrerer Hilfsstoffe, bevorzugt bis zu 10 Gew.-%, bevorzugter weniger als 5 Gew.-% eines oder mehrerer Hilfsstoffe, bezogen auf die Gesamtzusammensetzung, enthalten.

Ein bevorzugter Hilfsstoff ist ausgewählt aus der Gruppe der Fette und Öle, insbesondere aus der Gruppe der kosmetischen Öle. Diese können in den erfindungsgemäßen Formkörpern bevorzugt in einer Menge von bis zu 50 Gew.-%, bevorzugter 25 Gew.-% enthalten sein.

Weitere bevorzugte Hilfsstoffe sind ausgewählt aus der Gruppe der pH-Einstellungsmittel, besonders bevorzugt ist hierbei Salzsäure, die gegebenenfalls zur Einstellung des pH-Wertes der Formkörper-Zusammensetzungen auf pH < 7, bevorzugt ≤ 6, bevorzugter ≤ 5, noch bevorzugter ≤ 4 verwendet wird. Weist die Zusammensetzung aus Wirkstoffen, Gerüstbildnern und gegebenenfalls weiteren Hilfsstoffen per se bereits einen erfindungsgemäß gewünschten pH-Wert < 7 auf, so kann auf die Zugabe von pH-Einstellungsmitteln selbstverständlich verzichtet werden.

Die Formkörper enthalten gegebenenfalls auch Reste von Wasser. Da die in den Formkörpern enthaltenen Wirkstoffe jedoch erfindungsgemäß gegen insbesondere Feuchtigkeit stabilisiert werden sollen, ist der Wassergehalt so gering wie möglich zu halten. Je nach Art des Wirkstoffes (hydrophil, hydrophob) kann der Wassergehalt bei bis zu 10 Gew.-%, bezogen auf die Gesamtzusammensetzung, liegen. Der Wassergehalt kann sich nach der Herstellung des Formkörpers durch Gefriertrocknung bei der Lagerung verändern, in der Regel erhöhen. Bevorzugt liegt der Wassergehalt des Formkörpers nach der Herstellung bei maximal 10 Gew.-%, bevorzugt bei weniger als 5 Gew.-%, bevorzugter bei weniger als 1 Gew.-%, wobei überraschend gefunden wurde, dass durch das erfindungsgemäße Verfahren zur Herstellung der erfindungsgemäßen Formkörper, das die Einstellung des pH-Wertes der Zusammensetzung auf einen pH-Wert < 7, bevorzugt ≤ 6, bevorzugter ≤ 5, noch bevorzugter ≤ 4 als maßgebliches Merkmal der Erfindung umfasst, die Restfeuchte beim Trocknen der Formkörper deutlich erniedrigt werden kann gegenüber bekannten Formkörpern, die ohne einen solchen pH-Einstellungsschritt hergestellt werden wie z. B. in der DE 10248314 offenbart. Durch die herstellungsbedingte niedrigere Restfeuchte der Formkörper kann eine zusätzliche Stabilisierung der Wirkstoffe erzielt werden.

Ein besonders bevorzugter Formkörper enthält:

- >50 Gew.-% eines oder mehrerer Wirkstoffe, ausgewählt aus der Gruppe der sauren Wirkstoffe mit einen pKs-Wert bei 25 °C von < 7, insbesondere saure Wirkstoffe wie z. B. Ascorbinsäure (Vitamin C) oder seine Derivate oder Salicylsäure oder seine Derivate wie Acetylsalicylsäure (ASS)
- ≤ 15 Gew.-% eines oder mehrerer Gerüstbildner, wobei Proteine ausgenommen sind, wobei mindestens ein Gerüstbildner Chitosan ist,
- ≤25 Gew.-% eines oder mehrerer Hilfsstoffe, wie insbesondere kosmetische Öle sowie pH-Einstellungsmittel wie Salzsäure, sowie -bis zu 10 Gew.-%, bevorzugt bis zu 5 Gew.-%, bevorzugter weniger als 1 Gew.-% Wasser,

mit der Maßgabe, dass der Formkörper keine Proteine als Gerüstbildner aufweist, und dass dessen 1 gewichtsprozentige Lösung oder Suspension in Wasser bei 20 °C einen pH Wert < 7 aufweist.

Bevorzugt weist der erfindungsgemäß verwendete Formkörper, wie z.B. der der vorstehend erwähnten Zusammensetzung, enthaltend > 50 Gew.-% eines oder mehrerer Wirkstoffe sowie maximal 15 Gew.-% eines oder mehrerer Gerüstbildner, wobei Proteine ausgenommen sind wobei mindestens ein Gerüstbildner Chitosan ist,

- einen pH-Wert ≤ 6, bevorzugter ≤ 5, noch bevorzugter ≤ 4, gemessen in dessen 1 gewichtsprozentiger Lösung oder Suspension in Wasser bei 20 °C,
- eine Dichte von 0,005 g/cm$^3$ bis zu 0,8 g/cm$^3$ bevorzugt 0,01 g/cm$^3$ bis zu 0,8 g/cm$^3$,
- ein Volumen von 0,1 cm$^3$ bis 6 cm$^3$, bevorzugt 0,8 cm$^3$ bis 6 cm$^3$,
- einen Durchmesser (maximaler Abstand zwischen zwei Punkten des Formkörpers) von mindestens 6 mm auf und/oder
- besitzt bevorzugt eine sphärische Ausgestaltung, besonders bevorzugt die Form einer Kugel.

Die erfindungsgemäßen Formkörper stellen poröse Formkörper mit homogener Verteilung der Inhaltsstoffe dar (abgesehen von gegebenenfalls vorhandenen Beschichtungen).

Die erfindungsgemäßen Formkörper wie beispielsweise solche wie vorstehend genannt, werden bevorzugt mit einer wässrigen Flüssigkeit / Aktivatorlösung aufgelöst, die enthält:

- mindestens 70 Gew.-% Wasser,
- mindestens 5 Gew.-% mehrwertige Alkohole,
- bis zu 10 Gew.-% eines oder mehrerer Wirkstoffe wie insbesondere solche aus der Gruppe der kosmetischen Wirkstoffe
- bis zu 20 Gew.-% eines oder mehrerer Hilfsstoffe, wie insbesondere solche aus der Gruppe der kosmetischen Öle, wie insbesondere Capryl/Capronsäure-Triglyceride und zweckmäßige Emulgatoren, und die einen pH-Wert von 5 - 7 und des weiteren einen Gehalt an Erdalkaliionen wie insbesondere Calcium- und/oder Magnesiumionen von weniger als 1 Gew-% aufweist.

Die Auflösungsgeschwindigkeit der erfindungsgemäß verwendeten Formkörper, gemessen entsprechend einer Methode zur Messung der "Zerfallszeit von Tabletten und Kapseln" mit einer Testapparatur nach PharmEU beträgt weniger als 30 Sekunden, noch bevorzugter weniger als 20 Sekunden (bei Formkörpern mit 11 mm Durchmesser liegt nach < 10 Sekunden komplette Hydratation ohne erkennbaren Kern vor).

Die erfindungsgemäßen Formkörper sind erhältlich durch ein Verfahren, das die folgenden Schritte umfasst:

(a) Herstellen einer wässrigen Lösung oder Suspension, die einen oder mehrere Gerüstbildner, wobei Proteine ausgenommen sind, wobei mindestens ein Gerüstbildner Chitosan ist, einen oder mehrerer Wirkstoffe, sowie gegebenenfalls einen oder mehrere Hilfsstoffe enthält,

(b) gegebenenfalls Einstellen des pH Werts dieser wässrigen Lösung oder Suspension auf pH < 7,

(c) Giessen der Mischung in eine Form,

(d) Gefrieren der Mischung in der Form und

(e) Gefriertrocknung der gefrorenen Mischung unter Bildung des Formkörpers.

Bevorzugt erfolgt die Einstellung des pH-Wertes der wässrigen Lösung oder Suspension in Schritt (b) auf einen pH-Wert von < 6, noch bevorzugter auf einen pH-Wert < 4.

Gegebenenfalls können zwischen diesen Schritten weitere Schritte durchgeführt werden, insbesondere ist es möglich, nach dem Schritt (d) die gefrorenen Formkörper aus der Form zu entnehmen. Weiterhin ist möglich, daraufhin eine Bearbeitung der Oberfläche der gefrorenen Formkörper durch mechanische Bearbeitung oder durch Aufbringen oder Besprühen mit z.B. Wirkstofflösungen, Farbstofflösungen und/oder die Auflösegeschwindigkeit modifizierenden Mitteln durchzuführen. Bevorzugt weist der Formkörper jedoch keine Oberflächenbeschichtung auf und ist homogen, im Sinne einer gleichen Verteilung der Bestandteile über den gesamten Formkörper aufgebaut.

Zweckmäßig geht man bei der Herstellung so vor, dass man zunächst eine wässrige Lösung des Gerüstbildners herstellt und anschließend den oder die Wirkstoffe sowie gegebenenfalls einen oder mehrere Hilfsstoffe hinzugibt und vermischt. Gegebenenfalls wird nach Vermischen aller Bestandteile gegebenenfalls durch Zugabe der Hilfsstoffe aus der Gruppe der pH-Einstellungsmittel der gewünschte pH-Wert auf < 7, bevorzugt ≤ 6, bevorzugter ≤ 5, noch bevorzugter ≤ 4 eingestellt.

Damit dem Formkörper eine ausreichende mechanische Stabilität verliehen werden kann, ist es erforderlich, dass die Lösung bzw. Suspension eine gewisse Konzentration des Gerüstbildners aufweist, die jedoch erfindungsgemäß maximal 15 Gew.-% bezogen auf die gefriergetrocknete Gesamtzusammensetzung beträgt. Die jeweilige genaue Konzentration hängt natürlich ab von der Art des verwendeten Gerüstbildners. Sie beträgt zweckmäßig etwa mindestens 0,3 Gew.-% bezogen auf die Gesamtmenge der Lösung, bevorzugt mindestens etwa 0,5 Gew.-% bis zu etwa 1,0 Gew.-%, bevorzugt weniger als 3,0 Gew.-%, noch bevorzugter weniger als 1,5 Gew.-% (Gewicht des Gerüstbildners bezogen auf das Gesamtgewicht der Lösung).

Die Menge der in der Lösung bzw. Suspension enthaltenden Feststoffe wie Gerüstbildner, Wirkstoffe und Hilfsstoffe beeinflusst maßgeblich die Dichte (Gewicht des Formkörpers bezogen auf das Volumen der geometrischen Form des Formkörpers) des erhaltenen Formkörpers. Die Dichte ist wiederum eine wichtige Größe für die Porosität des Formkörpers und damit wiederum für die Auflösungsgeschwindigkeit des Formkörpers beim Befeuchten mit Wasser oder einer Wirk- und/oder Hilfsstofflösung. Die poröse Struktur gefriergetrockneter Formkörper ist eine wesentliche Grundlage für die schnelle Löslichkeit, da durch die große Oberfläche im porösen Material ein inniger Austausch zwischen wässriger Phase und festem Formkörper während des Rehydratisierungsprozesses stattfinden kann. Je höher die Konzentration der Wirkstoffe, des Gerüstbildners sowie ggf. der Hilfsstoffe in der Lösung ist, umso höher wird die Dichte und damit umso geringer wird der Porositätsgrad des Formkörpers und umgekehrt. Allerdings hängt der Porositätsgrad der Formkörper nicht allein von der Materialdichte ab. Vielmehr ist die Materialporosität im wesentlichen eine Funktion zweier Parameter, der Materialdichte und der Eiskristallgröße. Hohe Feststoffgehalte in der wässrigen Suspension erhöhen die Materialdichte im gefriergetrockneten Endprodukt und verringern die Kontaktfläche Rehydratisierungsmittel/Feststoff. Hohe Einfriergradienten führen zu kleinen Eiskristallen, welche zu großen inneren Materialoberflächen führen, was wiederum die Rehydratisierung begünstigt. Zur schnellen Befeuchtung und Auflösung der gefriergetrockneten Formkörper sind also niedrige Materialdichten und kleine Eiskristalle vorteilhaft.

Verwendet man nun in der Emulsion hohe Wirkstoffkonzentrationen, welche aufgrund ihrer elektrolytischen Dissoziation den Gefrierpunkt des Wassers herabsetzen wie z.B. Ascorbinsäure (Vitamin C) oder Acetylsalicylsäure, so bilden sich in der Regel große Eiskristalle und es treten generelle Probleme im Gefrierprozess auf. Ein hoher Anteil nicht einfrierbaren Wassers mit hohen Wirkstoffkonzentrationen ist die Folge, welches als Konsequenz in der Gefriertrocknung zu einem partiellen Strukturkollaps des gefriergetrockneten Endproduktes führt. Der Formkörper "taut an".

Unter dem Gesichtspunkt Dichte/Porositätsgrad bzw. der Auflösungsgeschwindigkeit wird die Rezepturierung und Herstellung der erfindungsgemäßen Formkörper so ausgerichtet, dass die Dichten der damit erhältlichen Formkörper zweckmäßig bei etwa 0,01 $g/cm^3$ bis zu 0,8 $g/cm^3$, bevorzugt etwa 0,015 $g/cm^3$ bis zu 0,5 $g/cm^3$.bevorzugt etwa 0,02 $g/cm^3$ bis zu 0,25 $g/cm^3$ liegen. Der Begriff der Dichte, wie er vorliegend verwendet wird, bezeichnet das Gewicht des Formkörpers bezogen auf das Volumen der äußeren geometrischen Form des Formkörpers.

Das Gewicht der einzelnen Formkörper hängt natürlich von deren Größe ab. Im allgemeinen liegt das Gewicht der einzelnen Formkörper bei etwa 10 bis 300 mg, bevorzugt 20 bis 200 mg. Beispielsweise weisen Kugeln von 11 mm Durchmesser ein Gewicht im Bereich von bevorzugt 20 bis 150 mg, bevorzugter 50 bis 125 mg auf. Für Kugeln anderer Durchmesser berechnen sich entsprechende Vorzugsbereiche.

Unter dem Gesichtspunkt der Eiskristallbildung/Porositätsgrad wurde überraschend gefunden, dass die Antauproblematik und die damit einhergehende Bildung von großen Eiskristallen mit hohem Anteil nicht einfrierbarem Wasser bei der Einarbeitung hoher Wirkstoffkonzentrationen insbesondere saurer Wirkstoffe mit dissoziierbaren Säure-/Basengruppen, einerseits durch Einstellen des pH-Werts der Lösung auf einen pH < 7, bevorzugt ≤ 6, bevorzugter ≤ 5, noch bevorzugter ≤ 4, um das Säure/Base-Gleichgewicht zugunsten der undissoziierten Säure zu verschieben, und andererseits durch Auswahl einer Einfriergeometrie bei der der Formkörper bei mindestens < -20 °C gleichzeitig von allen Seiten eingefroren wird, gelöst werden kann. Dieses Verfahren, Frieren innerhalb einer Form beispielsweise durch Anblasen mit kalter Luft, reduziert den Anteil nicht ausfrierbaren Wassers auf ein Minimum, wodurch anschließend bei höheren Temperaturen getrocknet werden kann, was wiederum die Kosten für den Gefriertrocknungsprozess erheblich senkt. Zusätzlich begünstigt eine sphärische Ausgestaltung wie insbesondere eine Kugelgeometrie die Trocknung der Formkörper mit hohen Wirkstoffgehalten, da durch das günstige Oberfläche/Volumen Verhältnis der Kugelform die Sublimation durch das Produkt nach allen Seiten hin symmetrisch und auf kleine Strecken aufgerichtet ist, wodurch wiederum der Dampftransport während der Sublimation erleichtert wird.

Die Herstellung der Lösung, die der Gefriertrocknung unterworfen wird, erfolgt bevorzugt so, dass zunächst eine Lösung des Gerüstbildners hergestellt wird und anschließend in die Lösung des Gerüstbildners die gegebenenfalls vorhandenen Wirkstoffe bzw. Hilfsstoffe eingearbeitet werden. Werden öllösliche Wirkstoffe verwendet, werden diese bevorzugt in gegebenenfalls als Hilfsstoffe verwendeten Ölen (insbesondere Squalan, Jojobaöl und Triglyceriden) gelöst und anschließend der Lösung des Gerüstbildners zugesetzt. Diese Herstellweise besitzt den Vorteil, dass sich kurzzeitig stabile Lösungen bzw. Suspensionen bilden. Es werden keine oder nur geringe Mengen an Emulgatoren oder oberflächenaktiven Substanzen wie z. B. Tenside benötigt, und es findet während der Verarbeitung keine Phasentrennung der Lösung oder Suspension bei Verwendung öllöslicher bzw. öliger Hilfs- bzw. Wirkstoffe statt. Bevorzugt werden jedoch wasserlösliche Wirkstoffe verwendet.

Die so hergestellte Lösung wird dann in Formen gegossen, die den Formkörpern entsprechende Hohlräume der gewünschten geometrischen Formen aufweisen. Die Form besteht bevorzugt aus Kautschuk, Silikon-Kautschuk, vulkanisiertem Kautschuk (Gummi) etc. Bevorzugt sind Gummiformen. Die Formmaterialien können gegebenenfalls beschichtet sein. Die Hohlräume der Formkörper, in die die Lösung hineingegossen wird, weisen im allgemeinen die Form des gewünschten Formkörpers auf. D.h., dass das Volumen des Hohlraums im wesentlichen dem Volumen der später erhaltenen Formkörper entspricht.

Da das Volumen der in die Hohlräume eingefüllten Lösungen bzw. Suspensionen beim Gefrieren zunimmt (Dichteunterschied zwischen Wasser und Eis), werden die Hohlräume in der Regel nicht vollständig gefüllt. Es werden auf diese Weise vollständig symmetrische Formkörper erhalten. Dies ist beispielsweise nach dem Verfahren durch Eintropfen in tiefkalte Lösungen (wie in flüssigen Stickstoff) nicht möglich, da es dort zu unsymmetrischen Temperaturverteilungen kommt, so dass sich stets mehr oder weniger starke Abweichungen von einer regelmäßigen Form ergeben. Solche unregelmäßig geformten Formkörper sind jedoch gerade im Bereich der kosmetischen Endprodukte nicht erwünscht. Das bedeutet in der Regel, dass diese nach dem Eintropfverfahren hergestellten Formkörper einer mechanischen Nachbearbeitung bedürfen, was nach dem Verfahren, wie es erfindungsgemäß verwendet wird, nicht erforderlich ist. Bei nach dem Eintropfverfahren hergestellten Formkörpern wird eine solche Nachbearbeitung mit zunehmendem Volumen des Formkörpers immer erforderlicher, da bei diesem Verfahren deutliche äußerliche Unregelmäßigkeiten auftreten, die insbesondere bei größeren Formkörpern stärker in Erscheinung treten.

Nach dem Einfüllen der Lösung in die Hohlräume der Form wird die Lösung bzw. Suspension gefroren. Das Abkühlen bzw. Gefrieren der Lösung kann an sich in beliebiger Weise erfolgen wie beispielsweise durch Anblasen mit kalter Luft, Abkühlen durch Aufbringen auf eine mit Kühlsole durchflossene Platte oder auch das Eintauchen der Formen in flüssige Gase, wie z.B. das Eintauchen in flüssigen Stickstoff. Die Abkühlgeschwindigkeit beeinflusst dabei die Größe der gebildeten Eiskristalle. Diese beeinflussen wiederum die Porengrößenverteilung des gebildeten Formkörpers. Werden wenige große Kristalle gebildet, so weist der Formkörper wenige große Poren auf, was aus besagten Gründen der Antauproblematik bei der Einarbeitung hoher Konzentrationen gefrierpunkterniedrigender Wirkstoffe nicht erwünscht ist. Werden viele kleine Kristalle gebildet, weist der Formkörper viele kleine Poren auf. Die Kristalle werden um so kleiner, je höher die Abkühlgeschwindigkeit der Lösung bzw. Suspension ist. Wie bereits ausgeführt wird eine Einfriergeometrie bevorzugt, bei der die Formkörper bei mindestens < -20 °C in der Form gleichzeitig von allen Seiten eingefroren werden.

Die Gefriertemperatur, die erforderlich ist, hängt unter anderem davon ab, wie stark die Gefrierpunktserniedrigung durch die in der Lösung enthaltenden Wirkstoffe bzw. Hilfsstoffe ist. Zweckmäßig liegt die Temperatur unterhalb des Gefrierpunktes von Wasser bis zur Temperatur von flüssigem Stickstoff (- 196°C). Bevorzugt ist die Gefriertemperatur etwa -20 bis -80°C, besonders bevorzugt -25 bis - 45 °C Nach dem Gefrieren der Lösung bzw. Suspension werden die Formkörper aus der Form genommen und gegebenenfalls einer Nachbearbeitung unterzogen. Die Nachbearbeitung kann mechanisch erfolgen, z.B. durch eine Oberflächenbehandlung (Schleifen, Aufrauhen etc.). Desweiteren ist eine Beschichtungsbehandlung möglich wie z.B. das Besprühen mit einer Salzlösung, z.B. zur Bildung weniger löslicher Formen der Gerüstbildner, insbesondere bei der Verwendung von Natriumalginat und Salzlösungen mehrwertiger Metallionen. Auch kann eine Farblösung oberflächlich auf die gefrorenen Formkörper aufgebracht werden, die zu gefärbten Formkörpern führt.

Anschließend werden die Formkörper der Gefriertrocknung unterworfen. Die Gefriertrocknung kann in an sich bekannter Weise nach allgemein bekannten Gefriertrocknungsverfahren, wie z.B. auch in der DE 4328329 C2, der DE 4028622C2 oder der DE 10350654 A1 beschrieben, erfolgen.

Die Erfindung umfasst insbesondere die folgenden bevorzugten Ausführungsformen:

1. Gefriergetrockneter Formkörper, dadurch gekennzeichnet, dass er > 50 Gew. % eines oder mehrerer Wirkstoffe und ≤ 15 Gew. % eines oder mehrerer Gerüstbildner, wobei Proteine ausgenommen sind, wobei mindestens ein Gerüstbildner Chitosan ist, sowie gegebenenfalls einen oder mehrere Hilfsstoffe, jeweils bezogen auf die Gesamtzusammensetzung des gefriergetrockneten Formkörpers, enthält, und dessen 1 gewichtsprozentige Lösung oder Suspension in Wasser bei 20 °C einen pH Wert < 7 aufweist.

2. Gefriergetrockneter Formkörper nach Ausführungsform 1 mit einem Wirkstoffanteil ≥ 75 Gew. %, bevorzugter ≥ 90 Gew. %, noch bevorzugter ≥ 95 Gew. % jeweils bezogen auf die Gesamtzusammensetzung des gefriergetrockneten Formkörpers.

3. Gefriergetrockneter Formkörper nach einer der Ausführungsformen 1 oder 2, worin eine 1 gewichtsprozentige Lösung oder Suspension des Wirkstoffs in Wasser bei 20 °C einen pH Wert < 7 aufweist.

4. Gefriergetrockneter Formkörper nach einer der Ausführungsformen 1 bis 3, worin der Wirkstoff ausgewählt ist aus der Gruppe der sauren Wirkstoffe mit einen pKs-Wert bei 25 °C von ≤ 7.

5. Gefriergetrockneter Formkörper nach einer der Ausführungsformen 1 bis 4, worin der Wirkstoff ausgewählt ist aus der Gruppe, die besteht aus Ascorbinsäure und deren Derivate, und Salicylsäure und deren Derivate, insbesondere Acetylsalicylsäure, Clofibrinsäure, Ibuprofen, Gemfibrozil, Fenoprofen, Naproxen, Ketoprofen, Indomethacin, Bezafibrat, Tolfenaminsäure, Diclofenac, Meclofenaminsäure, Paracetamol, Acitretin, Acrivastin, Azelainsäure, Cromolyn, Ethacrynsäure, Furosemid, Penicillin und Derivate davon, Vitamin A und Derivate davon, Risedronsäure und Derivate davon, Liponsäure, und Ursodiol.

6. Gefriergetrockneter Formkörper nach einer der Ausführungsformen 1 bis 5, der bezogen auf die Gesamtzusammensetzung des gefriergetrockneten Formkörpers > 50 Gew. % eines Wirkstoffs aus der Gruppe der Ascorbinsäure und ihrer Derivate enthält.

7. Gefriergetrockneter Formkörper nach einer der Ausführungsformen 1 bis 5, der bezogen auf die Gesamtzusammensetzung des gefriergetrockneten Formkörpers > 50 Gew.% eines Wirkstoffs aus der Gruppe der Salicylsäure und deren Derivate, bevorzugt aus der Gruppe der Acetylsalicylsäure und ihrer Derivate enthält.

8. Gefriergetrockneter Formkörper nach einer der Ausführungsformen 1 bis 7 mit einem Gerüstbildneranteil ≤ 10 Gew. %, bevorzugter ≤ 5 Gew. %, jeweils bezogen auf die Gesamtzusammensetzung des gefriergetrockneten Formkörpers.

10. Gefriergetrockneter Formkörper nach Ausführungsform 9, worin mindestens ein Gerüstbildner Chitosan ist, wobei Proteine ausgenommen sind.

13. Gefriergetrockneter Formkörper nach einer der Ausführungsformen 1 bis 12 dessen 1 gewichtsprozentige Lösung oder Suspension in Wasser bei 20 °C einen pH Wert ≤ pH 6,0, bevorzugt ≤ pH 5,0, bevorzugter ≤ pH 4,0 aufweist.

14. Gefriergetrockneter Formkörper nach einer der Ausführungsformen 1 bis 13, dessen 1 gewichtsprozentige Lösung oder Suspension in Wasser bei 20 °C einen pH Wert < pH 6,0 aufweist und worin der Wirkstoff ausgewählt ist aus der Gruppe der sauren Wirkstoffe mit einen pKs-Wert bei 25 °C von ≤ 7.

15. Gefriergetrockneter Formkörper nach einer der Ausführungsformen 1 bis 14, dessen 1 gewichtsprozentige Lösung oder Suspension in Wasser bei 20 °C einen pH Wert < pH 4,0 aufweist und worin der Wirkstoff ausgewählt ist aus der Gruppe der sauren Wirkstoffe mit einen pKs-Wert bei 25 °C von ≤ 7.

16. Gefriergetrockneter Formkörper nach einer der Ausführungsformen 1 bis 13, mit einem Gerüstbildneranteil < 15 Gew. % bezogen auf die Gesamtzusammensetzung des gefriergetrockneten Formkörpers und worin der Wirkstoff ausgewählt ist aus der Gruppe der sauren Wirkstoffe mit einen pKs-Wert bei 25 °C von ≤ 7.

17. Gefriergetrockneter Formkörper nach einer der Ausführungsformen 1 bis 16 der bei Flüssigkeitszufuhr in ≤ 30 Sekunden vollständig zerfällt.

18. Gefriergetrockneter Formkörper nach einer der Ausführungsformen 1 bis 17 der ein Volumen von 0,1 cm$^3$ bis 6 cm$^3$, eine Dichte von 0,01 g/cm$^3$ bis 0,8 g/ cm$^3$ und/oder die geometrische Form einer Kugel mit einem Durchmesser von mindestens 6 mm aufweist.

19.Verfahren zur Herstellung eines gefriergetrockneten Formkörpers gemäß einer der Ausführungsformen 1 bis 18 das die Schritte umfasst:

(a) Herstellen einer wässrigen Lösung oder Suspension, die einen oder mehrere Gerüstbildner, wobei Proteine ausgenommen sind, wobei mindestens ein Gerüstbildner Chitosan ist, einen oder mehrerer Wirkstoffe, sowie gegebenenfalls einen oder mehrere Hilfsstoffe enthält,
(b) gegebenenfalls Einstellen des pH Werts dieser wässrigen Lösung oder Suspension auf pH < 7,
(c) Giessen der Mischung in eine Form,
(d) Gefrieren der Mischung in der Form und
(e) Gefriertrocknung der gefrorenen Mischung unter Bildung des Formkörpers.

20. Verfahren nach Ausführungsform 19, worin die gefrorene Mischung aus Schritt (d) vor der Gefriertrocknung gemäß Schritt (e) aus der Form genommen wird.

21. Verfahren nach einer der Ausführungsformen 19 oder 20, worin der pH Wert in Schritt (b) auf pH < 6, bevorzugter auf pH < 4 eingestellt wird.

22. Gefriergetrockneter Formkörper erhältlich nach dem Verfahren nach einer der Ausführungsformen 19 bis 21.

23. Verwendung des gefriergetrockneten Formkörpers nach einer der Ausführungsformen 1 bis 18 oder 22 als kosmetisches Mittel.

24. Verwendung des gefriergetrockneten Formkörpers nach einer der Ausführungsformen 1 bis 18 oder 22 als pharmazeutisches Mittel.

25. Verwendung nach Ausführungsform 23 oder 24, worin die Anwendung äußerlich erfolgt.

26. Verwendung nach einer der Ausführungsformen 23 bis 25, worin der gefriergetrocknete Formkörper mit Wasser oder einer wässrigen Lösung eines oder mehrerer Wirkstoffe und/oder gegebenenfalls Hilfsstoffe angefeuchtet wird und zerfällt und anschließend auf die Haut oder auf das Haar aufgetragen wird.

27. Verwendung des gefriergetrockneten Formkörpers nach einer der Ausführungsformen 1 bis 18 oder 22 zur oralen oder peroralen Applikation von Wirkstoffen.

28. Kit-of-parts Kombination enthaltend mindestens einen gefriergetrockneten Formkörper gemäß einer der Ausführungsformen 1 bis 18 oder 22 sowie mindestens eine wässrige Lösung, die einen oder mehrere Wirkstoffe und/oder gegebenenfalls einen oder mehrere Hilfsstoffe enthält, in zusammengehöriger, räumlicher Anordnung.

29. Verwendung der Kit-of-parts Kombination nach Ausführungsform 28 als kosmetisches Mittel.

30. Verwendung der Kit-of-parts Kombination nach Ausführungsform 28 als therapeutisches Mittel.

31. Verwendung nach einer der Ausführungsformen 23 bis 27 und 29 bis 30, die direkt durch den Endverbraucher erfolgt.

Die Erfindung wird des weiteren durch die folgenden Beispiele näher veranschaulicht.

BEISPIELE

Vergleichsbeispiel **1 (Ascorbinsäurekugel, Durchmesser 11 mm)**

<div align="center">

Ascorbinsäure mit Hyaluronsäure

| | |
|---|---|
| 1,0 g | Hyaluronsäure |
| 16,0 g | Ascorbinsäure |
| 83,0 g | Wasser |

</div>

1,0 g Hyaluronsäure werden unter Rühren in 83,0 g Wasser gegeben und homogen gemischt. Anschließend wird unter Rühren 16,0 g Ascorbinsäure hinzugegeben, dabei wird die Mischung, die einen pH-Wert ≤ 3,0 aufweist, auf einer Temperatur von 0-10 °C gehalten. Die homogene (entgaste) Mischung wird in Formen gegossen, unter Anblasen von Luft ausgefroren, aus der Form genommen und anschließend in an sich bekannter Weise gefriergetrocknet.

<div align="center">

Der gefriergetrocknete Formkörper enthält:

| | |
|---|---|
| 94,1 Gew.-% | Ascorbinsäure |
| 5,9 Gew.-% | Hyaluronsäure |

</div>

Die Auflösegeschwindigkeit der gefriergetrockneten Formkörper, gemessen nach einer Methode zur Messung der "Zerfallszeit von Tabletten und Kapseln" mit einer Testapparatur nach PharmEU beträgt weniger als 10 Sekunden.

**Beispiel 2 (Ascorbinsäurekugel, Durchmesser 11 mm)**

Eine Mischung wie in Vergleichsbeispiel 1 wird vor dem Einfüllen in Formen mit verdünnter Natronlauge (0,1 mol/L) auf pH 3,0 eingestellt.

Die Auflösegeschwindigkeit der gefriergetrockneten Formkörper, gemessen nach einer Methode zur Messung der "Zerfallszeit von Tabletten und Kapseln" mit einer Testapparatur nach PharmEU beträgt weniger als 5 Sekunden.

**Beispiel 3 (Ascorbinsäurekugel, Durchmesser 13 mm)**

<div align="center">

Ascorbinsäure mit Chitosan

| | |
|---|---|
| 0,9 g | Chitosan |
| 17,6 g | Ascorbinsäure |
| 81,5 g | Wasser |

</div>

0,9 g Chitosan werden unter Rühren in 81,5 g Wasser gegeben und homogen gemischt. Das Chitosan wird durch Zugabe von konz. Salzsäure in Lösung gebracht und ein pH-Wert von 3,0 nach Auflösen des Chitosans eingestellt. Anschließend wird unter Rühren 17,6 g Ascorbinsäure hinzugegeben, dabei wird die Mischung, die einen pH-Wert < 3 aufweist, auf einer Temperatur von 0-10 °C gehalten. Die homogene (entgaste) Mischung wird in Formen gegossen, unter Anblasen von Luft ausgefroren, aus der Form genommen und anschließend in an sich bekannter Weise gefriergetrocknet.

<div align="center">

Der gefriergetrocknete Formkörper enthält:

| | |
|---|---|
| 95,1 Gew.-% | Ascorbinsäure |
| 4,8 Gew.-% | Chitosan |

</div>

Die Auflösegeschwindigkeit der gefriergetrockneten Formkörper, gemessen nach einer Methode zur Messung der "Zerfallszeit von Tabletten und Kapseln" mit einer Testapparatur nach PharmEU beträgt weniger als 10 Sekunden.

**Beispiel 4 (Ascorbinsäurekugel, Durchmesser 13 mm)**

Eine Mischung wie in Beispiel 3 wird vor dem Einfüllen in Formen mit verdünnter Natronlauge (0,1 mol/L) auf pH 3 eingestellt.

Die Auflösegeschwindigkeit der gefriergetrockneten Formkörper, gemessen nach einer Methode zur Messung der "Zerfallszeit von Tabletten und Kapseln" mit einer Testapparatur nach PharmEU beträgt weniger als 10 Sekunden.

Vergleichsbeispiel **5 (Ascorbinsäurekugel mit Alginat + Carboxymethylcellulose + Hyaluronsäure, Durchmesser 15 mm)**

| | |
|---|---|
| 0,5 g | Na-Alginat |
| 0,5 g | Natrium-Hyaluronat |
| 0,5 g | Natrium-Carboxymethylcellulose |
| 9,0 g | Ascorbinsäure |
| 89,5 g | Wasser |

0,5 g Natrium Alginat, 0,5 g Natrium-Hyaluronat und 0,5 g NatriumCarboxymethylcellulose werden unter Rühren in 89,5 g Wasser gegeben und homogen gemischt. Anschließend wird unter Rühren 9,0 g Ascorbinsäure hinzugegeben, dabei wird die Mischung, die einen pH-Wert < 3 aufweist, auf einer Temperatur von 0-10 °C gehalten. Die homogene (entgaste) Mischung wird in Formen gegossen, unter Anblasen von Luft ausgefroren, aus der Form genommen und anschließend in an sich bekannter Weise gefriergetrocknet.

| | |
|---|---|
| Der gefriergetrocknete Formkörper enthält: | |
| 85,7 Gew.-% | Ascorbinsäure |
| 4,8 Gew.-% | Hyaluronsäure |
| 4,8 Gew.-% | Na-Alginat |
| 4,8 Gew.-% | Carboxymethylcellulose |

Die Auflösegeschwindigkeit der gefriergetrockneten Formkörper, gemessen nach einer Methode zur Messung der "Zerfallszeit von Tabletten und Kapseln" mit einer Testapparatur nach PharmEU beträgt weniger als 10 Sekunden.

Vergleichsbeispiel **6 (Acetylsalicylsäure mit Alginat, Durchmesser 9 mm)**

| | |
|---|---|
| 0,8 g | Na-Alginat |
| 0,3 g | Carrageenan |
| 5,0 g | Neutralöl |
| 4,0 g | PEG-40 Hydrogenated Castor oil |
| 13,0 g | Acetylsalicylsäure |
| 76,9 g | Wasser |

0,8 g Natrium Alginat, 0,3 g Carrageenan werden unter Rühren in 76,9 g Wasser gegeben und homogen gemischt. 13 g Acetylsalicylsäure werden in 5 g Neutralöl und 4 g PEG-40 Hydrogenated Castor oil aufgeschlämmt. Die Aufschlämmung wird unter Rühren mit der Alginat/Carrageenan-Suspension vereinigt. Anschließend wird die Mischung mit verdünnter Salzsäure auf einen pH-Wert < 3 eingestellt. Die homogene (entgaste) Mischung wird in Formen gegossen, unter Anblasen von Luft ausgefroren, aus der Form genommen und anschließend in an sich bekannter Weise gefriergetrocknet.

| | |
|---|---|
| Der gefriergetrocknete Formkörper enthält: | |
| 56,3 % | Acetylsalicylsäure |
| 21,6 % | Neutralöl |

(fortgesetzt)

| | |
|---|---|
| 17,3 % | PEG-40 Hydrogenated Castor oil |
| 3,5 % | Na-Alginat |
| 1,3 % | Carrageenan |

Die Auflösegeschwindigkeit der gefriergetrockneten Formkörper, gemessen nach einer Methode zur Messung der "Zerfallszeit von Tabletten und Kapseln" mit einer Testapparatur nach PharmEU beträgt weniger als 10 Sekunden.

**Beispiel 7 (Acetylsalicylsäurekugel + Chitosan, Durchmesser 9 mm)**

| | |
|---|---|
| 1,0 g | Chitosan |
| 5,0 g | Neutralöl |
| 4,0 g | PEG-40 Hydrogenated Castor oil |
| 13,0 g | Acetylsalicylsäure |
| 77,0 g | Wasser |

1,0 g Chitosan werden unter Rühren unter Zugabe konzentrierter Salzsäure in 77,0 g Wasser gegeben gelöst. Die Chitosan-Lösung wird auf einen pH-Wert von 3,0 gebracht. 13 g Acetylsalicylsäure werden in 5 g Neutralöl und 4 g PEG-40 Hydrogenated Castor oil aufgeschlämmt. Die Aufschlämmung wird unter Rühren mit der Chitosan-Suspension vereinigt. Anschließend wird die Mischung auf einen pH-Wert $\leq 3$ eingestellt. Die homogene (entgaste) Mischung wird in Formen gegossen, unter Anblasen von Luft ausgefroren, aus der Form genommen und anschließend in an sich bekannter Weise gefriergetrocknet.

Der gefriergetrocknete Formkörper enthält:

| | |
|---|---|
| 56,5 % | Acetylsalicylsäure |
| 21,8 % | Neutralöl |
| 17,4 % | PEG-40 Hydrogenated Castor oil |
| 4,3 % | Chitosan |

Die Auflösegeschwindigkeit der gefriergetrockneten Formkörper, gemessen nach einer Methode zur Messung der "Zerfallszeit von Tabletten und Kapseln" mit einer Testapparatur nach PharmEU beträgt weniger als 5 Sekunden.

Vergleichsbeispiel **8 (Ascorbinsäurekugel + kationisierte Stärke,** Durchmesser 11 mm)

Ascorbinsäure mit kationisierter Stärke

| | |
|---|---|
| 2,0 g | kationisierte Stärke |
| 16,0 g | Ascorbinsäure |
| 82,0 g | Wasser |

2,0 g kationisierte Stärke werden unter Rühren in 82,0 g Wasser gegeben und homogen gemischt. Anschließend wird unter Rühren 16,0 g Ascorbinsäure hinzugegeben, dabei wird die Mischung, die einen pH-Wert $\leq 3,0$ aufweist, auf einer Temperatur von 0-10 °C gehalten. Die homogene (entgaste) Mischung wird in Formen gegossen, unter Anblasen von Luft ausgefroren, aus der Form genommen und anschließend in an sich bekannter Weise gefriergetrocknet.

Der gefriergetrocknete Formkörper enthält:

| | |
|---|---|
| 11,1 Gew.-% | kationisierte Stärke |
| 88,9 Gew.-% | Ascorbinsäure |

Die Auflösegeschwindigkeit der gefriergetrockneten Formkörper, gemessen nach einer Methode zur Messung der "Zerfallszeit von Tabletten und Kapseln" mit einer Testapparatur nach PharmEU beträgt weniger als 5 Sekunden.

**Patentansprüche**

1.  Gefriergetrockneter Formkörper, **dadurch gekennzeichnet, dass** er
    > 50 Gew. % eines oder mehrerer Wirkstoffe, ausgewählt aus der Gruppe der sauren Wirkstoffe mit einen pKs-Wert bei 25 °C von ≤ 7, und
    ≤ 15 Gew. % eines oder mehrerer Gerüstbildner, wobei Proteine ausgenommen sind, wobei mindestens ein Gerüstbildner Chitosan ist,
    sowie gegebenenfalls
    einen oder mehrere Hilfsstoffe, jeweils bezogen auf die Gesamtzusammensetzung des gefriergetrockneten Formkörpers, enthält, und dessen 1 gewichtsprozentige Lösung oder Suspension in Wasser bei 20 °C einen pH Wert < 7 aufweist.

2.  Gefriergetrockneter Formkörper nach Anspruch 1 mit einem Wirkstoffanteil ≥ 75 Gew. %, bevorzugter ≥ 90 Gew. %, noch bevorzugter ≥ 95 Gew. % jeweils bezogen auf die Gesamtzusammensetzung des gefriergetrockneten Formkörpers.

3.  Gefriergetrockneter Formkörper nach einem der Ansprüche 1 bis 2, worin die Wirkstoffe ausgewählt sind aus der Gruppe umfassend Ascorbinsäure und ihre Derivate die aus der Gruppe die Glycoside, Ester und deren Alkalimetall-Erdalkalimetall- und trivalenten Metallsalze ausgewählt sind, und Salicylsäure und deren Derivat die Acetylsalicylsäure.

4.  Gefriergetrockneter Formkörper nach einem der Ansprüche 1 bis 3 mit einem Gerüstbildneranteil ≤ 10 Gew. %, bevorzugter ≤ 5 Gew. %, jeweils bezogen auf die Gesamtzusammensetzung des gefriergetrockneten Formkörpers.

5.  Gefriergetrockneter Formkörper nach einem der Ansprüche 1 bis 4 dessen 1 gewichtsprozentige Lösung oder Suspension in Wasser bei 20 °C einen pH Wert < 4,0 aufweist.

6.  Gefriergetrockneter Formkörper nach einem der Ansprüche 1 bis 5, der bei Flüssigkeitszufuhr in ≤ 30 Sekunden vollständig zerfällt.

7.  Verfahren zur Herstellung eines gefriergetrockneten Formkörpers gemäß einem der Ansprüche 1 bis 6 das die Schritte umfasst:

    (a) Herstellen einer wässrigen Lösung oder Suspension, die einen oder mehrere Gerüstbildner, wobei Proteine ausgenommen sind, einen oder mehrerer Wirkstoffe, sowie gegebenenfalls einen oder mehrere Hilfsstoffe enthält, wobei mindestens ein Gerüstbildner Chitosan ist.
    (b) gegebenenfalls Einstellen des pH Werts dieser wässrigen Lösung oder Suspension auf pH < 7,
    (c) Giessen der Mischung in eine Form,
    (d) Gefrieren der Mischung in der Form und
    (e) Gefriertrocknung der gefrorenen Mischung unter Bildung des Formkörpers.

8.  Gefriergetrockneter Formkörper nach einem der Ansprüche 1 bis 6 oder erhältlich nach dem Verfahren nach Anspruch 7 zur Verwendung als pharmazeutisches Mittel.

9.  Gefriergetrockneter Formkörper nach einem der Ansprüche 1 bis 6 oder erhältlich nach dem Verfahren nach Anspruch 7 zur Verwendung zur oralen oder peroralen Applikation von Wirkstoffen.

10. Verwendung eines gefriergetrockneten Formkörpers nach einem der Ansprüche 1 bis 6 oder erhältlich nach dem Verfahren nach Anspruch 7 als kosmetisches Mittel.

11. Verwendung nach Anspruch 10, worin der gefriergetrocknete Formkörper mit Wasser oder einer wässrigen Lösung eines oder mehrerer Wirkstoffe und/oder gegebenenfalls Hilfsstoffe angefeuchtet wird und zerfällt und anschließend auf die Haut oder auf das Haar aufgetragen wird.

12. Kit-of-parts Kombination enthaltend mindestens einen gefriergetrockneten Formkörper gemäß einem der Ansprüche 1 bis 6, 8, 9 oder erhältlich nach dem Verfahren nach Anspruch 7 sowie mindestens eine wässrige Lösung, die einen oder mehrere Wirkstoffe und/oder gegebenenfalls einen oder mehrere Hilfsstoffe enthält, in zusammengehöriger, räumlicher Anordnung.

**Claims**

1. Freeze-dried molded article **characterized in that** it comprises
   > 50% by wt. of one or more active substances selected from the group of acid active substances having a pKa value of < or equal to 7 at 25°C, and
   < or equal to 15% by wt. of one or more scaffold-forming agents, with proteins being excepted, as well as optionally one or more auxiliary substances, in each case based on the total composition of the freeze-dried molded article, and whose 1% by wt. solution or suspension in water, at 20 °C., has a pH value of < 7.

2. Freeze-dried molded article according to claim 1 comprising a content of acid active substances of > or equal to 75% by wt., preferably > or equal to 90% by wt., more preferably > or equal to 75% by wt., based on the total composition of the freeze-dried molded article.

3. Freeze-dried molded article according to claim 1 and 2, wherein the active substance is selected from the group comprising ascorbic acid and its derivatives selected from the group of glycosides, ester and its alkaline metal-, alkaline earth metal- and trivalent metal salts, and from the group of salicylic acid and its derivative acetylsalicylic acid.

4. Freeze-dried molded article according to claim 1 to 3, with a content of scaffold-forming agents of < or equal to 10% by wt., preferably < or equal to 10% by wt., more preferably < or equal to 5 % by wt., based on the total composition of the freeze-dried molded article.

5. Freeze-dried molded article according to claim 1 to 4, whose 1% by wt. solution or suspension in water has a pH value of <pH 4.0 at 20° C.

6. Freeze-dried molded article according to claim 1 to 5, which decomposes completely within $\leq$ 30 seconds when liquid is added.

7. Method for producing a freeze-dried molded article according to claim 1 to 6, comprising the following steps:

   (a) preparing an aqueous solution or suspension comprising one or more scaffold-forming agents, with proteins being excepted, one or more active substances, as well as one or more auxiliary substances, wherein at least one of the scaffold forming agents is chitosan,
   (b) optionally adjusting the pH value of this aqueous solution or suspension to pH <7,
   (c) pouring the mixture into a mold,
   (d) freezing the mixture in the mold, and
   (e) freeze drying the frozen mixture while forming the molded article.

8. Freeze-dried molded article according to claim 1 to 6 or that can be obtained in accordance with the method of claim 8, for use as pharmaceutical agent.

9. Freeze-dried molded article according to claim 1 to 6 or that can be obtained in accordance with the method of claim 8, for use in the oral or peroral application of active substances.

10. Use of a freeze-dried molded article according to claim 1 to 6 or that can be obtained in accordance with the method of claim 8, as cosmetic agent.

11. Use according to claim 10, wherein the freeze dried molded article is wetted with water or an aqueous solution of one or more active substances and/or, if applicable, auxiliary substances, and dissolves and is subsequently applied to the skin or to the hair.

12. Kit-of-parts combination containing at least one freeze-dried molded article according to one of the claims 1 to 6, 8, 9 or obtained by a method according to claim 7, as well as an aqueous solution of one or more active substances and/or, if applicable, one or more auxiliary substances, in conjugated spatial arrangement.

**Revendications**

1. Article moulé lyophilisé **caractérisé en ce qu'**il comprend
> 50 % p/p d'une ou plusieurs substances actives, choisies du groupe des substances actives acides avec un pKs à 25°C de ≤ 7, et
≤ 15 % p/p d'une ou plusieurs matières de structuration, où les protéines sont exclues, et où au moins une matière de structuration est le chitosane,
ainsi qu'éventuellement un ou plusieurs adjuvants,
dans chaque cas par rapport au poids total de la composition de l'article moulé lyophilisé, et où une solution ou suspension de 1 % duquel dans de l'eau à 20°C a un pH de < 7.

2. L'article moulé lyophilisé selon la revendication 1 avec une part de substance active de ≥ 75 % p/p, de préférence ≥ 90 % p/p, plus préférablement ≥ 95 % p/p dans chaque cas par rapport au poids total de la composition de l'article moulé lyophilisé.

3. L'article moulé lyophilisé selon l'une quelconque des revendications 1 à 2, où les substances actives sont choisies du groupe comprenant l'acide ascorbique et ses dérivés qui sont choisis du groupe des glycosides, des esters et de leurs sels de métaux alcalins, de métaux alcalino-terreux et de métaux trivalents, et de l'acide salicylique et de son dérivé l'acide acétylsalicylique.

4. L'article moulé lyophilisé selon l'une quelconque des revendications 1 à 3 avec une part de matière de structuration ≤ 10 % p/p, de préférence ≤ 5 % p/p, dans chaque cas par rapport au poids total de la composition de l'article moulé lyophilisé.

5. L'article moulé lyophilisé selon l'une quelconque des revendications 1 à 4, où une solution ou suspension de 1 % duquel dans de l'eau à 20°C a un pH de < 4,0.

6. L'article moulé lyophilisé selon l'une quelconque des revendications 1 à 5 qui se décompose complètement en ≤ 30 secondes lors de l'apport d'un liquide.

7. Procédé de fabrication d'un article moulé lyophilisé selon l'une quelconque des revendications 1 à 6 qui comprend les étapes :

   (a) fabriquer une solution ou suspension aqueuse qui comprend une ou plusieurs matières de structuration, où les protéines sont exclues, une ou plusieurs substances actives, ainsi qu'éventuellement un ou plusieurs adjuvants, où au moins une matière de structuration est le chitosane,
   (b) éventuellement ajuster le pH de ladite solution ou suspension aqueuse à < 7,
   (c) couler le mélange dans un moule,
   (d) congeler le mélange dans le moule et
   (e) lyophiliser le mélange congelé pour former l'article moulé.

8. L'article moulé lyophilisé selon l'une quelconque des revendications 1 à 6 ou obtenu par un procédé selon la revendication 7 pour l'utilisation en tant que préparation pharmaceutique.

9. L'article moulé lyophilisé selon l'une quelconque des revendications 1 à 6 ou obtenu par le procédé selon la revendication 7 pour l'utilisation dans l'application orale ou perorale.

10. Utilisation d'un article moulé lyophilisé selon l'une quelconque des revendications 1 à 6 ou obtenu par le procédé selon la revendication 7 en tant que composition cosmétique.

11. L'utilisation selon la revendication 10, dans laquelle l'article moulé lyophilisé est mouillé avec une solution aqueuse d'une ou plusieurs substances actives et/ou éventuellement adjuvants et se décompose et est ensuite appliqué à la peau ou aux cheveux.

12. Kit comprenant au moins un article moulé lyophilisé selon l'une quelconque des revendications 1 à 6, 8, 9 ou obtenu par un procédé selon la revendication 7 ainsi qu'au moins une solution aqueuse qui comprend une ou plusieurs substances actives et/ou éventuellement un ou plusieurs adjuvants dans un agencement spatial commun.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5405616 A
- DE 4201179
- US 5843347 A
- WO 05073296 A
- JP 2004149468 A
- DE 10248314
- WO 2004035023 A

- DE 2017373
- DE 102006038629
- WO 2008020066 A
- FR 2886845
- EP 0888769 A
- DE 4328329 C2
- DE 4028622 C2
- DE 10350654 A1

**In der Beschreibung aufgeführte Nicht-Patentliteratur**

- CTFA-Abhandlung, Cosmetic Ingredient Handbook. The Cosmetic, Toiletry and Fragrance Association, Inc, 1988